(19) Europäisches Patentamt / European Patent Office / Office européen des brevets

(11) **EP 3 919 104 B1**

(12) **EUROPEAN PATENT SPECIFICATION**

(45) Date of publication and mention of the grant of the patent:
**21.02.2024  Bulletin 2024/08**

(21) Application number: **21174238.2**

(22) Date of filing: **18.05.2021**

(51) International Patent Classification (IPC):
**A61M 15/08** *(2006.01)*    **G16H 20/00** *(2018.01)*

(52) Cooperative Patent Classification (CPC):
**A61M 15/08; G16H 20/13;** A61M 11/005;
A61M 11/042; A61M 15/0085; A61M 2205/13;
A61M 2205/276; A61M 2205/332; A61M 2205/3592;
A61M 2205/50; A61M 2205/505; A61M 2205/52;
A61M 2205/581; A61M 2205/582; A61M 2205/583;

(Cont.)

(54) **NASAL DROP DEVICE, NASAL DROP SUPPORT DEVICE, NASAL DROP SYSTEM, NASAL DROP ADMINISTRATION METHOD, SUPPORT METHOD, OPERATION METHOD OF NASAL DROP SYSTEM, AND PROGRAM**

NASENTROPFENVORRICHTUNG, NASENTROPFENUNTERSTÜTZUNGSVORRICHTUNG, NASENTROPFENSYSTEM, NASENTROPFENVERABREICHUNGSVERFAHREN, UNTERSTÜTZUNGSVERFAHREN, BETRIEBSVERFAHREN DES NASENTROPFENSYSTEMS UND PROGRAMM

DISPOSITIF DE GOUTTES NASALES, DISPOSITIF DE SUPPORT DE GOUTTES NASALES, SYSTÈME DE GOUTTES NASALES, PROCÉDÉ D'ADMINISTRATION DE GOUTTES NASALES, PROCÉDÉ DE SUPPORT, PROCÉDÉ DE FONCTIONNEMENT DU SYSTÈME DE GOUTTES NASALES ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.06.2020   JP 2020095390**

(43) Date of publication of application:
**08.12.2021   Bulletin 2021/49**

(60) Divisional application:
**24150084.2 / 4 322 172**

(73) Proprietor: **Funai Electric Co., Ltd.**
**Daito,**
**Osaka, 574-0013 (JP)**

(72) Inventors:
• **TANABE, Hideki**
  **Osaka, 574-0013 (JP)**
• **YONEKAWA, Ryo**
  **Osaka, 574-0013 (JP)**
• **MOTOBAYASHI, Naoki**
  **Osaka, 574-0013 (JP)**
• **AZUMA, Akihiro**
  **Osaka, 574-0013 (JP)**

(74) Representative: **Becker, Eberhard**
  **Becker Kurig & Partner**
  **Patentanwälte mbB**
  **Bavariastraße 7**
  **80336 München (DE)**

(56) References cited:
**WO-A1-2019/155150    WO-A1-2021/014076**

(52) Cooperative Patent Classification (CPC): (Cont.)
A61M 2205/587; A61M 2205/6018;
A61M 2205/8206

**Description**

BACKGROUND

Technical Field

The disclosure relates to a nasal drop support device, a nasal drop system, a support method, and a program.

Related Art

National Publication of International Patent Application No. 2020-501715 discloses an administration device assembly that includes a transnasal fluid administration device, a first facial support portion supported by the forehead or the nasal bridge of the user in operation, and a second facial support portion suitable for being supported by the upper lip of the user. Furthermore, Patent literature 1 raises an issue that the mounting angle and the insertion depth of a nozzle into the nasal cavity affect the administration efficiency, and describes that a vertical insertion angle of 40 to 50° and an insertion depth of 5 to 15 mm are appropriate.

National Publication of International Patent Application No. 2017-535396 does not disclose a nasal drop device, but discloses that an accelerometer is used in an inhaler for taking medicine by mouth to determine whether the user has correctly swung the inhaler as a preparatory work for inhalation.

National Publication of International Patent Application No. 3-501222 discloses a device that has an angle setting device for tilting the head of a patient by a predetermined angle such as 60° or the like from an upright posture, and allows a drug solution or a washing solution to reach the entire mucous membrane in the nasal cavity.

WO2019155150A1 proposes an assembly for dispensing a fluid product, comprising: a mechanism for dispensing a fluid product for the nose, said mechanism comprising a reservoir containing a fluid product, a dispensing head being mounted on said reservoir, and said dispensing head being provided with a dispensing opening; as well as a remote mobile device, such as a smartphone; in which said dispensing mechanism comprises at least one orientation sensor and communicates with said remote mobile device in order to assist and guide the user in real time so as to obtain an optimum orientation of the dispensing mechanism in the moment of the actuation, said orientation sensor, such as an accelerometer or a gyroscope, determines the spatial orientation of said mechanism in real time, particularly the angle of said device in relation to said remote mobile appliance, said mechanism comprises a second sensor, such as an accelerometer, for detecting when the mechanism is actuated by the user, and said remote mobile device comprises an accelerometer which allows the determination of the spatial position thereof, particularly the angle of said remote mobile device in relation to the vertical.

WO2021014076A1 proposes Assembly for dispensing a fluid product, comprising: a nasal fluid product dispenser, the nasal dispenser comprising a reservoir containing the fluid product, a dispensing member for dispensing a dose of fluid product on each actuation, and a dispensing head assembled on the dispensing member, the dispensing head being provided with a dispensing orifice, a remote mobile device attached to the user's head, such as an earpiece, the nasal device and the remote mobile device attached to the user's head each comprising an orientation sensor, such as an accelerometer or a gyroscope, the orientation sensors cooperating to determine the position of the nasal dispenser in the user's nostril, the nasal dispenser comprising an indicator, which is visible to the user when the nasal dispenser is inserted into a nostril, to guide the user towards an optimal orientation of the nasal dispenser in the nostril, and the nasal dispenser comprising an electronic module and automatic actuation means designed to automatically actuate the nasal device when the orientation sensors detect that the nasal dispenser is arranged in an optimal position in the nostril.

SUMMARY

Conventionally, drugs for central nervous system diseases such as Alzheimer's disease are mainly transdermally administered, which is inefficient and requires a large amount of drugs. As a method for improving efficiency, a method of directly administering drugs to the central nervous system from the nasal cavity has been devised. In order for the nasal drops to function optimally, administration to a specific site in the nasal cavity is necessary. Here, the specific site is, for example, a site in the nasal cavity called the olfactory region.

In a conventional nasal drop device, an appropriate amount is difficult to administer to a specific site in an ejection direction, the drug solution is administered in a manner that the amount of the drug solution to be administered at one time is larger than the originally required amount, and the drug solution tends to be wasted. It is not easy to accurately match the ejection direction of the drug solution from the nasal drop device to the specific site to be administered to, and if the drug solution deviates from the part where the drug is desired to be administered, the administration efficiency deteriorates. As a result, the effective administration amount may be lower than expected. Therefore, it is necessary to apply the nasal cavity device to the nose at an appropriate angle in order to accurately administer an appropriate amount of the drug solution to the specific site.

The present invention is provided by the appended claims. The following disclosure serves a better understanding of the present invention. The disclosure provides a nasal drop device, a nasal drop support device, and the like that can simply and easily administer an appropriate amount of solution to a specific site in the nasal cavity without waste.

In order to achieve the above object, the nasal drop device according to an aspect of the disclosure includes: an ejection portion that ejects droplets into the nasal cavity; and a control portion that acquires suitability information indicating whether or not a current relative angle between a reference line indicating the facial posture of a user who puts the nasal drop device in the nose and a reference line indicating the posture of the nasal drop device is suitable for the nasal drop, and controls the ejection operation of the ejection portion according to the suitability information.

Accordingly, the nasal drop can be performed when the relative angle is appropriate, but not performed when the relative angle is not appropriate, and an appropriate amount of solution can be simply and easily administered to a specific site in the nasal cavity without waste.

For example, the nasal drop device may further include a wireless portion for wireless communication. The control portion may acquire the suitability information from another device via the wireless portion.

**[0013]** [0012]Accordingly, because the suitability information is acquired from another device via the wireless portion, an increase in the circuit scale and the circuit cost can be suppressed.

For example, the nasal drop device may further include an acceleration sensor that detects a tilt angle of the nasal drop device with respect to a vertical direction. The wireless portion may transmit the tilt angle detected by the acceleration sensor to the another device.

Accordingly, precision of the relative angle can be further improved, and the administration of an appropriate amount of solution to a specific site can be made simple and easy.

For example, the nasal drop device may further include a camera that captures an image of the face of the user who puts the nasal drop device in the nose. The control portion may calculate the relative angle by analyzing the image captured by the camera, determine whether or not the deviation degree between the relative angle and a target angle is within an allowable range, and acquire the determination result as the suitability information.

Accordingly, the relative angle can be calculated by analyzing the image, the acceleration sensor may not be included, and the increase in the circuit scale and the circuit cost can be suppressed.

For example, the nasal drop device may further include an acceleration sensor that detects a tilt angle of the nasal drop device with respect to a vertical direction. The control portion may calculate the relative angle by analyzing the image captured by the camera based on the tilt angle.

Accordingly, precision of the relative angle can be further improved, and the administration of an appropriate amount of solution to a specific site can be made simple and easy.

For example, in the nasal drop device, the ejection portion may eject droplets of several picolitres to several tens of picolitres in a piezoelectric way or a thermal way.

Accordingly, the amount of the ejected liquid can be controlled with a fine precision in the picolitre order, and the administration of an appropriate amount can be facilitated.

For example, the nasal drop device may further include a notification portion that notifies the user of the content of the suitability information by using at least one of light, sound, and vibration.

Accordingly, the appropriate use of the nasal drop device by the user can be supported. For example, if the suitability information indicates appropriateness, the operation of the user to start the nasal drop can be prompted, and if the suitability information indicates inappropriateness, the user can be prompted to adjust the angle of the nasal drop device with respect to the face of the user.

For example, in the control device, when the suitability information indicates appropriateness, the control portion may cause the ejection portion to start the ejection operation without waiting for an operation instructing start of the nasal drop from the user.

Accordingly, the nasal drop can be performed without missing the time during which the appropriate current relative angle is maintained, that is, before the current appropriate relative angle deviates.

In addition, a nasal drop support device according to an aspect of the disclosure is a nasal support device that supports use of a nasal drop device by a user and includes: a camera that captures an image of the face of the user who puts the nasal drop device in the nose; a processor that calculates, based on the image captured by the camera, a current relative angle between a reference line indicating the facial posture of the user and a reference line indicating the posture of the nasal drop device, and generates suitability information indicating whether or not the deviation degree between the relative angle and a target angle is within an allowable range; and a notification portion that notifies the user of the content of the suitability information.

Accordingly, an appropriate amount of nasal drop to a specific site can be supported without missing the time during which the appropriate current relative angle is maintained, that is, before the current appropriate relative angle deviates.

For example, the nasal drop support device may further include a communication portion that transmits the suitability information to the nasal drop device.

Accordingly, the nasal drop device can be controlled in a manner that the nasal drop is not performed when the above relative angle is not appropriate and the nasal drop is performed when the above relative angle is appropriate, and an appropriate amount of solution can be simply and easily administered to a specific site in the nasal cavity without waste.

For example, the nasal drop support device may further include an acceleration sensor that detects a tilt angle of the camera with respect to a vertical direction as first angle information. The communication portion may receive, from the nasal drop device, second angle information indicating a tilt angle of the nasal drop device with respect to the vertical direction. The processor may detect, as third angle information, a tilt angle between the reference line indicating the facial posture of the user and a reference line indicating the posture of the nasal drop support device, by analyzing the image captured by the camera and the first angle information, and may calculate the relative angle based on the second angle information and the third angle information.

Accordingly, precision of the relative angle can be further improved, and the administration of an appropriate amount of solution to a specific site can be made simple and easy.

For example, the nasal drop support device may further include a memory that stores in advance individual data indicating, as a target, a relative angle between a reference line indicating the facial posture of each user and the reference line of the nasal drop device, and general-purpose data indicating, as an average target, a relative angle between reference lines indicating facial postures of a plurality of users and the reference line indicating the posture of the nasal drop device. The processor may select one of the individual data and the general-purpose data, and determines the target angle from the selection result.

Accordingly, an appropriate amount of solution can be simply and easily administered to a specific site in the nasal cavity of the user according to the individual data without waste, and the same effect can also be achieved according to the general-purpose data.

For example, the nasal drop support device may be a mobile device.

Accordingly, the mobile device already owned by the user can be used as a nasal drop support device 200, and the cost burden on the user can be suppressed.

For example, the nasal drop support device 200 may further include a notification portion that notifies the user of the content of the suitability information by using at least one of light, sound, and vibration.

Accordingly, an appropriate amount of nasal drop can be easily achieved for a specific site without missing the time at which the appropriate current relative angle is maintained, that is, before the current appropriate relative angle deviates.

In addition, a nasal drop system according to an aspect of the disclosure includes: a nasal drop device that ejects droplets into the nasal cavity; and a nasal drop support device that supports use of the nasal drop device by a user. One of the nasal drop support device and the nasal drop device has an acquisition portion that acquires an image obtained by capturing an image of the face of the user who puts the nasal drop device in the nose. One of the nasal drop support device and the nasal drop device has a processor that calculates, based on the image, a current relative angle between a reference line indicating the facial posture of the user and a reference line indicating the posture of the nasal drop device, and generates suitability information indicating whether or not the deviation degree between the relative angle and a target angle is within an allowable range. One of the nasal drop support device and the nasal drop device has a notification portion that notifies the user of the content of the suitability information.

Accordingly, by the notification, the nasal drop can be performed when the relative angle is appropriate, but not performed when the relative angle is not appropriate, and an appropriate amount of solution can be simply and easily administered to a specific site in the nasal cavity without waste.

For example, one of the nasal drop support device and the nasal drop device may have a camera that generates the image obtained by capturing an image of the face of the user who puts the nasal drop device in the nose. The acquisition portion may acquire the image from the camera. The processor may recognize the face of the user and the nasal drop device from the image, specify the reference line indicating the facial posture of the user and the reference line indicating the posture of the nasal drop device from the recognition result, and calculate the relative angle from the specification result.

Accordingly, the relative angle can be calculated by analyzing the image, the acceleration sensor may not be included, and the increase in the circuit scale and the circuit cost can be suppressed.

For example, at least one of the nasal drop support device and the nasal drop device may have an acceleration sensor that detects a tilt angle with respect to a vertical direction, and the processor may calculate the relative angle by using the tilt angle detected by the acceleration sensor.

Accordingly, precision of the relative angle can be further improved, and the administration of an appropriate amount of solution to a specific site can be made simple and easy.

For example, the nasal drop support device may include the acquisition portion, the processor, and the camera.

Accordingly, the nasal drop support device can be configured based on a mobile device such as a smartphone or the like. In addition, the nasal drop support device can be configured based on a camera device having a wireless communication function.

For example, the nasal drop device may include a first acceleration sensor that serves as the acceleration sensor, and the nasal drop support device may include the acquisition portion, the processor, the camera, and a second acceleration sensor that serves as the acceleration sensor.

Accordingly, precision of the relative angle can be further improved, and the administration of an appropriate amount of solution to a specific site can be made simple and easy.

A nasal drop administration method according to an aspect of the disclosure that is a nasal drop administration method of a nasal drop device that ejects droplets into the nasal cavity, in which suitability information is acquired that indicates whether or not a current relative angle between a reference line indicating the facial posture of a user who puts the nasal drop device in the nose and a reference line indicating the posture of the nasal drop device is suitable for the nasal drop; and the ejection operation is controlled according to the suitability information.

Accordingly, the nasal drop can be performed when the relative angle is appropriate, but not performed when the relative angle is not appropriate, and an appropriate amount of solution can be simply and easily administered to a specific site in the nasal cavity without waste.

A nasal drop support method according to an aspect of the disclosure is a support method of a nasal drop support device that supports use of a nasal drop device by a user, in which an image of the face of the user who puts the nasal drop device in the nose is captured by a camera; a current relative angle between a reference line indicating the facial posture of the user and a reference line indicating the posture of the nasal drop device is calculated based on the image captured by the camera; suitability information that indicates whether or not the deviation degree between the relative angle and a target angle is within an allowable range is generated; and the content of the suitability information is notified to the user.

Accordingly, an appropriate amount of nasal drop to a specific site can be supported without missing the time during which the appropriate current relative angle is maintained, that is, before the current appropriate relative angle deviates.

An operation method of a nasal drop system according to an aspect of the disclosure is an operation method of a nasal drop system including a nasal drop device that ejects droplets into the nasal cavity and a nasal drop support device that supports use of the nasal drop device by a user, in which one of the nasal drop support device and the nasal drop device acquires an image obtained by capturing an image of the face of the user who puts the nasal drop device in the nose; one of the nasal drop support device and the nasal drop device calculates, based on the image, a current relative angle between a reference line indicating the facial posture of the user and a reference line indicating the posture of the nasal drop device; one of the nasal drop support device and the nasal drop device generates suitability information indicating whether or not the deviation degree between the relative angle and a target angle is within an allowable range; and one of the nasal drop support device and the nasal drop device notifies the user of the content of the suitability information.

Accordingly, by the notification, the nasal drop can be performed when the relative angle is appropriate, but not performed when the relative angle is not appropriate, and an appropriate amount of solution can be simply and easily administered to a specific site in the nasal cavity without waste.

A program of a nasal drop device according to an aspect of the disclosure is executed in a computer included in a nasal drop device that ejects droplets into the nasal cavity, and is executed to: acquire suitability information that indicates whether or not a current relative angle between a reference line indicating the facial posture of a user who puts the nasal drop device in the nose and a reference line indicating the posture of the nasal drop device is suitable for the nasal drop; and control the ejection operation according to the suitability information.

Accordingly, the nasal drop can be performed when the relative angle is appropriate, but not performed when the relative angle is not appropriate, and an appropriate amount of solution can be simply and easily administered to a specific site in the nasal cavity without waste.

A program of a nasal drop support device according to an aspect of the disclosure is executed in a computer included in a nasal drop support device that supports use of a nasal drop device by a user, and is executed to: capture an image of the face of the user who puts the nasal drop device in the nose by a camera; calculate, based on the image captured by the camera, a current relative angle between a reference line indicating the facial posture of the user and a reference line indicating the posture of the nasal drop device; generate suitability information indicating whether or not the deviation degree between the relative angle and a target angle is within an allowable range; and notify the user of the content of the suitability information.

Accordingly, an appropriate amount of nasal drop to a specific site can be supported without missing the time during which the appropriate current relative angle is maintained, that is, before the current appropriate relative angle deviates.

A program of a nasal drop system according to an aspect of the disclosure is executed in a computer included in a nasal drop system including a nasal drop device that ejects droplets into the nasal cavity and a nasal drop support device that supports use of the nasal drop device by a user, and the program is executed in order that: one of the nasal drop support device and the nasal drop device acquires an image obtained by capturing an image of the face of the user who puts the nasal drop device in the nose; one of the nasal drop support device and the nasal drop device calculates, based on the image, a current relative angle between a reference line indicating the facial posture of the user and a reference line indicating the posture of the nasal drop device; one of the nasal drop support device and the nasal drop device generates suitability information indicating whether or not the deviation degree between the relative angle and a target angle is within an allowable range; and one of the nasal drop support device and the nasal drop device notifies the user of the content of the suitability information.

Accordingly, by the notification, the nasal drop can be performed when the relative angle is appropriate, but not performed when the relative angle is not appropriate, and an appropriate amount of solution can be simply and easily administered to a specific site in the nasal cavity without waste.

Moreover, the disclosure can be achieved not only in the form of a nasal drop device including a control portion that executes the above-mentioned characteristic processing, but also in the form of a method making the characteristic processing included in the nasal drop device as steps.

Similarly, the disclosure can be achieved not only in the form of a nasal drop support device including a processor that executes the above-mentioned characteristic processing, but also in the form of a method making the characteristic processing included in the nasal drop support device as steps.

Furthermore, the disclosure can be achieved not only in the form of a nasal drop system including a processor that executes the above-mentioned characteristic processing, but also in the form of a method making the processing performed by the characteristic processor included in the nasal drop system as steps.

In addition, the disclosure can also be achieved in the form of a program that makes a computer function to perform the characteristic control included in the nasal drop device, or in the form of a program that makes a computer execute the characteristic steps included in the nasal drop administration method.

Similarly, the disclosure can also be achieved in the form of a program that makes a computer function to perform the characteristic control included in the nasal drop support device, or in the form of a program that makes a computer execute the characteristic steps included in the nasal drop support method.

Furthermore, the disclosure can also be achieved in the form of a program that makes a computer function to perform the characteristic control included in the nasal drop system, or in the form of a program that makes a computer execute the characteristic steps included in the operation method of the nasal drop system.

Besides, it is evident that the program can be distributed via a computer-readable non-temporary recording medium such as a compact disc-read only memory (CD-ROM), or a communication network such as the Internet.

According to the nasal drop device, the nasal drop support device and the like of the disclosure, an appropriate amount of solution can be simply and easily administered to a specific site in the nasal cavity without waste.

BRIEF DESCRIPTION OF THE DRAWINGS

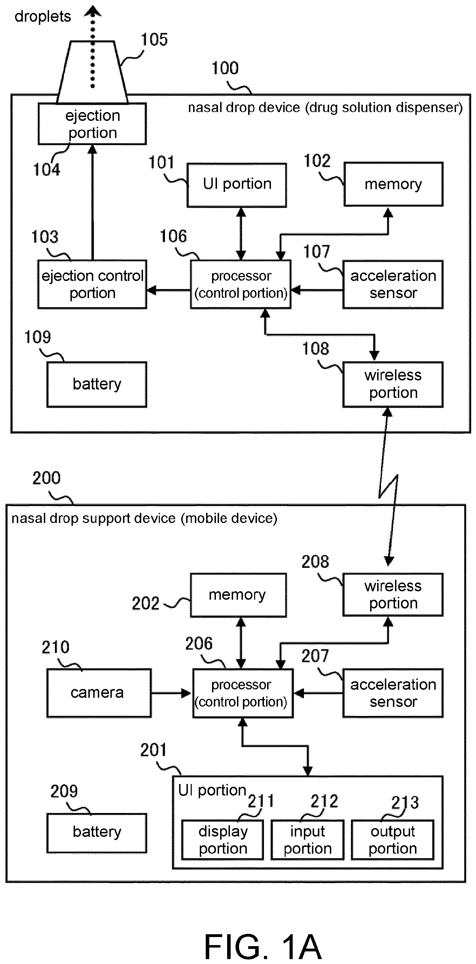

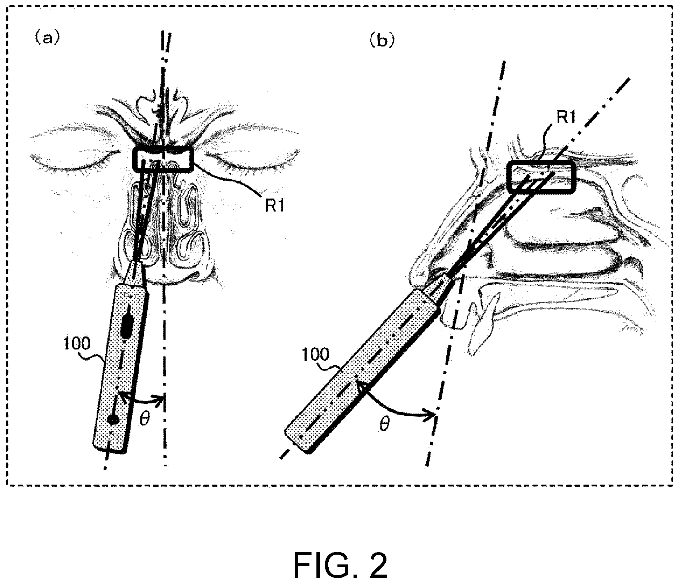

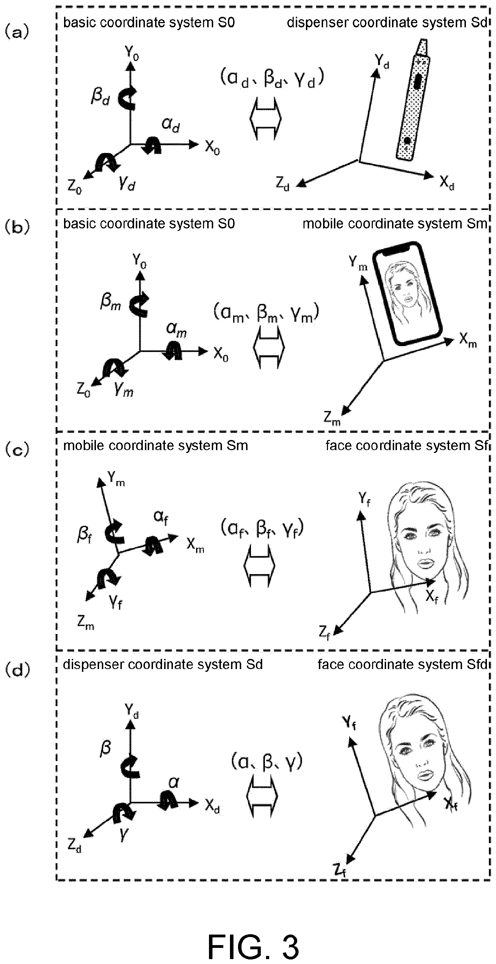

FIG. 1A is a block diagram showing a configuration example of a nasal drop system according to Embodiment 1.

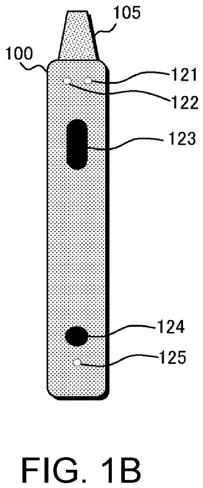

FIG. 1B is a block diagram showing an appearance example of a nasal drop device according to Embodiment 1.
(a) of FIG. 2 and (b) of FIG. 2 is an illustration diagram showing a state in which the nose drop device according to Embodiment 1 is put in the nose of a user.
(a) ~ (d) of FIG. 3 is an illustration diagram showing tilt angles and coordinate systems of the nasal drop device, a nasal drop support device, and the face of the user according to Embodiment 1.
FIG. 4 is a flowchart showing an operation example of the nasal drop system according to Embodiment 1.
FIG. 5 is a block diagram showing a configuration example of a nasal drop system according to Embodiment 2 (not according to the invention).
FIG. 6 is a flowchart showing an operation example of the nasal drop system according to Embodiment 2 (not according to the invention).
FIG. 7 is a block diagram showing a configuration example of a nasal drop system according to Embodiment 3.
FIG. 8 is a flowchart showing an operation example of the nasal drop system according to Embodiment 3.
FIG. 9 is a block diagram showing a configuration example of a nasal drop system according to Embodiment 4 (not according to the invention).
FIG. 10 is a flowchart showing an operation example of the nasal drop system according to Embodiment 4 (not according to the invention).
FIG. 11A is a block diagram showing a configuration example of a nasal drop device according to Embodiment 5.
FIG. 11B is a block diagram showing an appearance example of the nasal drop device according to Embodiment 5.

FIG. 12 is a flowchart showing an operation example of the nasal drop device according to Embodiment 5.

FIG. 13 is a block diagram showing a configuration example of a nasal drop device according to Embodiment 6 (not according to the invention).

FIG. 14 is a flowchart showing an operation example of the nasal drop device according to Embodiment 6 (not according to the invention).

FIG. 15 is a block diagram showing a configuration example of a nasal drop system according to Embodiment 7.

FIG. 16 is a block diagram showing a configuration example of a nasal drop system according to Embodiment 8.

DESCRIPTION OF THE EMBODIMENTS

Hereinafter, embodiments of the disclosure are described in detail with reference to the drawings. Moreover, all of the embodiments described below are comprehensive or specific examples. Numerical values, shapes, materials, components, arrangement positions and connection forms of the components, steps, the order of steps, and the like shown in the following embodiments are examples, and are not intended to limit the disclosure. In addition, among the components in the following embodiments, components not described in the independent claims will be described as arbitrary components.

(Embodiment 1)

[Configuration of nasal drop system]

FIG. 1A is a block diagram showing a configuration example of a nasal drop system 1 according to Embodiment 1. FIG. 1B is a block diagram showing an appearance example of a nasal drop device 100 according to Embodiment 1. (a) of FIG. 2 and (b) of FIG. 2 is an illustration diagram showing a state in which the nose drop device according to Embodiment 1 is put in the nose of a user.

The nasal drop system 1 shown in FIG. 1A includes the nasal drop device 100 and a nasal drop support device 200.

The nasal drop device 100 is a small pen-type device and is also called a drug solution dispenser. The nasal drop device 100 acquires suitability information indicating whether or not a current relative angle between a reference line indicating the facial posture of the user who puts the nasal drop device 100 in the nose and a reference line indicating the posture of the nasal drop device 100 is suitable for nasal drop, and performs an ejection operation of an ejection portion according to the suitability information.

Here, the reference line indicating the facial posture of the user refers to, for example, a straight line connecting the midpoint of the line segment connecting the pupils of both eyes and the center of the lips in a three-dimensional image obtained by capturing an image of the face of the user. The one-dot chain lines in (a) of FIG. 2 and (b) of FIG. 2 show examples of the reference line indicating the facial posture of the user. Moreover, the reference line indicating the facial posture may be a straight line connecting the midpoint of the line segment connecting the pupils of both eyes and the apex of the nose, or may be a central axis in a vertical direction of the upright head.

In addition, the reference line indicating the posture of the nasal drop device 100 is, for example, a central axis in an ejection direction of the droplets. The two-dot chain line in (a) of FIG. 2 and (b) of FIG. 2 show examples of the reference line indicating the posture of the nasal drop device 100. Moreover, the reference line indicating the posture of the nasal drop device 100 may be a ridge line among ridge lines of the outer shape of the nasal drop device 100, which substantially coincides with the ejection direction of the droplets.

In addition, the above relative angle is expressed as, for example, a rotation angle in a three-dimensional coordinate system. In (a) of FIG. 2 and (b) of FIG. 2, the relative angle $\theta$ between the one-dot chain line and the two-dot chain line is represented by $(\alpha, \beta, \gamma)$.

The nasal drop support device 200 calculates the current relative angle between the reference line indicating the facial posture of the user and the reference line indicating the posture of the nasal drop device 100, generates suitability information indicating whether or not a deviation degree between the relative angle and a target angle is within an allowable range, and transmits the suitability information to the nasal drop device 100. In (a) of FIG. 2 and (b) of FIG. 2, a region R1 surrounded by a solid line indicates an olfactory region as a specific site for the drug administration in the nasal cavity. In the examples of (a) of FIG. 2 and (b) of FIG. 2, the ejection direction indicated by the reference line shown by the one-dot chain line faces the region R1, and thus the relative angle in the diagram is also the most suitable target angle for the nasal drops.

Next, a configuration example of the nasal drop device 100 is described.

Therefore, the nasal drop device 100 includes a user interface (UI) portion 101, a memory 102, an ejection control portion 103, an ejection portion 104, a nose piece 105, a processor 106, an acceleration sensor 107, a wireless portion 108, and a battery 109.

The UI portion 101 receives the user operation and notifies the user of the operation state of the nasal drop device 100 and the like. In the appearance example of the nasal drop device 100 shown in FIG. 1B, the UI portion 101 includes LEDs 121 and 122, an operation button 123, a power button 124, and a power LED 124. For example, the LED 121 can selectively emit three colors of RGB, and notifies the user of the operation state of the nasal drop device 100 by lighting on, blinking, and lighting off. The same applies to the LED 122. The operation button 123 is a button for instructing start of the nasal drop operation. The power button 124 is a button for operating power on/off of the nasal drop device 100. The power LED 124 notifies the state of charge of the battery 109 during a power-on period. Moreover, the UI portion 101 may include a vibrator for transmitting vibration to the user, a buzzer or a speaker for transmitting sound to the user, or a liquid crystal display panel capable of displaying characters.

The memory 102 includes, for example, a ROM, a RAM, an electrically erasable flash memory, and the like, and stores a program executed by the processor 106 and various data.

The ejection control portion 103 controls the ejection operation of the ejection portion 104. For example, the ejection control portion 103 controls start and end of the ejection operation, and controls the number of times of the droplet ejection, an ejection interval time, and the like in the ejection operation. Because one droplet is in the picolitre order, the total amount of liquid to be ejected can be finely adjusted through the number of times of the ejection.

The ejection portion 104 ejects droplets of several picolitres to several tens of picolitres in a piezoelectric way or a thermal way. The ejection portion 104 may have the same configuration as the main part of the head of a piezoelectric or thermal inkjet type printer.

The nose piece 105 is removable from the main body of the nasal drop device 100 and is inserted into the entrance of the nostril by the user. The nose piece 105 is made of, for example, silicone rubber and is selected from a plurality of types having different sizes or different shapes according to the size and the shape of the nasal cavity of the user.

The processor 106 is a control portion that controls the entire nasal drop device 100, and is specifically configured as a microcomputer that executes the program stored in the memory 102.

The acceleration sensor 107 is, for example, a sensor that detects angular acceleration in three axis directions of xyz for detecting a tilt angle of the nasal drop device 100 with respect to a vertical direction. Moreover, the acceleration sensor 107 may be a sensor that detects angular acceleration in one axis direction. In this case, the angular acceleration in the three axis directions can be detected by processing of decomposing the detected value in the three axis directions.

The wireless portion 108 is a circuit that wirelessly communicates with the nasal drop support device 200. The wireless portion 108 performs wireless communication by, for example, Bluetooth (registered trademark) and a wireless LAN compliant with the IEEE 802.11 standard.

The battery 109 is a secondary battery that supplies electric power to the nasal drop device 100, for example, a lithium-ion battery.

Next, a configuration example of the nasal drop support device 200 is described.

The nasal drop support device 200 includes a UI portion 201, a memory 202, a processor 206, an acceleration sensor 207, a wireless portion 208, a battery 209, and a camera 210. The hardware configuration of the nasal drop support device 200 may be the same as that of a smartphone. Moreover, the hardware configuration of the nasal drop support device 200 is not limited to the smartphone, and may be the same as that of a tablet device and a laptop computer.

The UI portion 201 includes a display portion 211, an input portion 212, and an output portion 213. The display portion 211 includes a liquid crystal display panel or an organic EL panel as a display panel. The input portion 212 includes a touch panel, operation buttons, a microphone and the like formed on the display panel of the display portion 211. The output portion 213 includes a speaker, an earphone jack, and the like.

The memory 202 includes, for example, a ROM, a RAM, an electrically erasable flash memory and the like, and stores a program executed by the processor 206 and various data. The various data stored in the memory 202 include individual data indicating, as a target, a relative angle between the reference line indicating the facial posture of each user and the reference line of the nasal drop device 100, and general-purpose data indicating, as an average target, a relative angle between reference lines indicating facial postures of a plurality of users and the reference line indicating the posture of the nasal drop device 100. The individual data is created by, for example, measuring an optimum relative angle for the shape or the size of the nasal cavity of the user of the nasal drop device 100 under the guidance of a doctor. The general-purpose data is used when the individual data of the user of the nasal drop device 100 has not been created.

The processor 206 is a control portion that controls the entire nasal drop support device 200. Specifically, the processor 206 is configured as a microcomputer that executes the program stored in the memory 202.

The acceleration sensor 207 is, for example, a sensor that detects angular acceleration in the three axis directions of xyz for detecting a tilt angle of the nasal drop support device 200 with respect to the vertical direction. Here, the tilt angle of the nasal drop support device 200 with respect to the vertical direction is the same as the tilt angle of the camera 210 with respect to the vertical direction. Moreover, the acceleration sensor 207 may be a sensor that detects angular acceleration in one axis direction. In this case, the angular acceleration in the three axis directions can be detected by processing of decomposing the detected value in the three axis directions.

The wireless portion 208 is a circuit that wirelessly communicates with the nasal drop device 100. The wireless portion 208 performs wireless communication by, for example, Bluetooth (registered trademark) and a wireless LAN compliant with the IEEE 802.11 standard.

The battery 209 is a secondary battery that supplies electric power to the nasal drop support device 200, for example, a lithium-ion battery.

The camera 210 captures an image of the face of the user who puts the nasal drop device 100 in the nose.

Next, the tilt angle detected by the acceleration sensors 107 and 207 and the corresponding coordinate system are described.

(a) ~ (d) of FIG. 3 is an illustration diagram showing tilt angles and coordinate systems of the nasal drop device, the nasal drop support device, and the face of the user according to Embodiment 1.

(a) of FIG. 3 shows a basic coordinate system S0, a dispenser coordinate system Sd, and a tilt angle ($\alpha_d$, $\beta_d$, $\gamma_d$). The basic coordinate system S0 is a gravitational coordinate system having axes that coincide with the vertical and horizontal directions. The dispenser coordinate system Sd is a coordinate system having an axis that coincides with or is orthogonal to the reference line indicating the posture of the nasal drop device 100. The tilt angle ($\alpha_d$, $\beta_d$, $\gamma_d$) indicates the tilt angle of the nasal drop device 100 detected by the acceleration sensor 107. In this case, ($X_d$ axis, $Y_d$ axis, $Z_d$ axis) of the dispenser coordinate system Sd is obtained by rotating ($X_0$ axis, $Y_0$ axis, $Z_0$ axis) of the basic coordinate system S0 by the tilt angle ($\alpha_d$, $\beta_d$, $\gamma_d$). The direction of the rotation is positive counterclockwise.

(b) of FIG. 3 shows the basic coordinate system S0, a mobile coordinate system Sm, and a tilt angle ($\alpha_m$, $\beta_m$, $\gamma_m$). The mobile coordinate system Sm is a coordinate system having an axis that coincides with or is orthogonal to the reference line of the nasal drop support device 200, which is a mobile device. The tilt angle ($\alpha_m$, $\beta_m$, $\gamma_m$) indicates the tilt angle of the nasal drop support device 200 detected by the acceleration sensor 207. ($X_m$ axis, $Y_m$ axis, $Z_m$ axis) of the mobile coordinate system Sm is obtained by rotating the ($X_0$ axis, $Y_0$ axis, $Z_0$ axis) of the basic coordinate system S0 by the tilt angle ($\alpha_m$, $\beta_m$, $\gamma_m$).

(c) of FIG. 3 shows the mobile coordinate system Sm, a face coordinate system Sf, and a tilt angle ($\alpha_f$, $\beta_f$, $\gamma_f$). The face coordinate system Sf is a coordinate system having an axis that coincides with or is orthogonal to the reference line indicating the facial posture of the user. The tilt angle ($\alpha_f$, $\beta_f$, $\gamma_f$) indicates a tilt angle of the face coordinate system Sf with respect to the mobile coordinate system Sm. ($X_f$ axis, $Y_f$ axis, $Z_f$ axis) of the face coordinate system Sf is obtained by rotating the ($X_m$ axis, $Y_m$ axis, $Z_m$ axis) of the mobile coordinate system Sm by the tilt angle ($\alpha_f$, $\beta_f$, $\gamma_f$).

(d) of FIG. 3 shows the dispenser coordinate system Sd, a face coordinate system Sfd, and a tilt angle ($\alpha_{fd}$, $\beta_{fd}$, $\gamma_{fd}$). The face coordinate system Sfd is a coordinate system having an axis that coincides with or is orthogonal to the reference line indicating the facial posture of the user. The tilt angle ($\alpha$, $\beta$, $\gamma$) indicates a tilt angle of the face coordinate system Sfd with respect to the dispenser coordinate system Sd. ($X_{fd}$ axis, $Y_{fd}$ axis, $Z_{fd}$ axis) of the face coordinate system Sfd is obtained by rotating the ($X_d$ axis, $Y_d$ axis, $Z_d$ axis) of the dispenser coordinate system Sd by a tilt angle ($\alpha$, $\beta$, $\gamma$). That is, the tilt angle ($\alpha$, $\beta$, $\gamma$) is a relative angle between the reference line indicating the facial posture of the user and the reference line indicating the posture of the nasal drop device 100.

The relationship between the tilt angles and the coordinate systems can be shown by equations (1) to (3).

[Formula 1]

$$\begin{pmatrix} X_d \\ Y_d \\ Z_d \end{pmatrix} = \begin{pmatrix} \cos\gamma_d & \sin\gamma_d & 0 \\ -\sin\gamma_d & \cos\gamma_d & 0 \\ 0 & 0 & 1 \end{pmatrix} \begin{pmatrix} \cos\beta_d & 0 & -\sin\beta_d \\ 0 & 1 & 0 \\ \sin\beta_d & 0 & \cos\beta_d \end{pmatrix} \begin{pmatrix} 1 & 0 & 0 \\ 0 & \cos\alpha_d & \sin\alpha_d \\ 0 & -\sin\alpha_d & \cos\alpha_d \end{pmatrix} \begin{pmatrix} X_0 \\ Y_0 \\ Z_0 \end{pmatrix} \quad (1)$$

$$\begin{pmatrix} X_m \\ Y_m \\ Z_m \end{pmatrix} = \begin{pmatrix} \cos\gamma_m & \sin\gamma_m & 0 \\ -\sin\gamma_m & \cos\gamma_m & 0 \\ 0 & 0 & 1 \end{pmatrix} \begin{pmatrix} \cos\beta_m & 0 & -\sin\beta_m \\ 0 & 1 & 0 \\ \sin\beta_m & 0 & \cos\beta_m \end{pmatrix} \begin{pmatrix} 1 & 0 & 0 \\ 0 & \cos\alpha_m & \sin\alpha_m \\ 0 & -\sin\alpha_m & \cos\alpha_m \end{pmatrix} \begin{pmatrix} X_0 \\ Y_0 \\ Z_0 \end{pmatrix} \quad (2)$$

$$\begin{pmatrix} X_f \\ Y_f \\ Z_f \end{pmatrix} = \begin{pmatrix} \cos\gamma_f & \sin\gamma_f & 0 \\ -\sin\gamma_f & \cos\gamma_f & 0 \\ 0 & 0 & 1 \end{pmatrix} \begin{pmatrix} \cos\beta_f & 0 & -\sin\beta_f \\ 0 & 1 & 0 \\ \sin\beta_f & 0 & \cos\beta_f \end{pmatrix} \begin{pmatrix} 1 & 0 & 0 \\ 0 & \cos\alpha_f & \sin\alpha_f \\ 0 & -\sin\alpha_f & \cos\alpha_f \end{pmatrix} \begin{pmatrix} X_m \\ Y_m \\ Z_m \end{pmatrix} \quad (3)$$

The tilt angle ($\alpha_d$, $\beta_d$, $\gamma_d$) of the dispenser coordinate system Sd is detected by the acceleration sensor 107 of the nasal drop device 100. In addition, the tilt angle ($\alpha_m$, $\beta_m$, $\gamma_m$) of the mobile coordinate system Sm is detected by the acceleration sensor 207 of the nasal drop support device 200.

As for the tilt angle ($\alpha_f$, $\beta_f$, $\gamma_f$) of the face coordinate system Sf, the tilt angle ($\alpha_f$, $\beta_f$, $\gamma_f$) of the face with respect to the camera 210 or the nasal drop support device 200 in the mobile coordinate system Sm is calculated from the face image of the user who puts the nose drop device in the nose, which is captured by the camera 210. In addition, the relative angle between the reference line indicating the facial posture of the user and the reference line indicating the posture of the nasal drop device 100, that is, the tilt angle ($\alpha$, $\beta$, $\gamma$) of the face coordinate system Sf is can be calculated by equations (2) to (3).

Moreover, in the following, the basic coordinate system S0, the dispenser coordinate system Sd, the mobile coordinate system Sm, the face coordinate system Sf and the face coordinate system Sfd may be respectively referred to as the SO-coordinate system, the Sd-coordinate system, the Sm-coordinate system, the Sf-coordinate system, and the Sfd-coordinate system.

The operation in the nasal drop system 1 according to Embodiment 1 configured as described above is described.

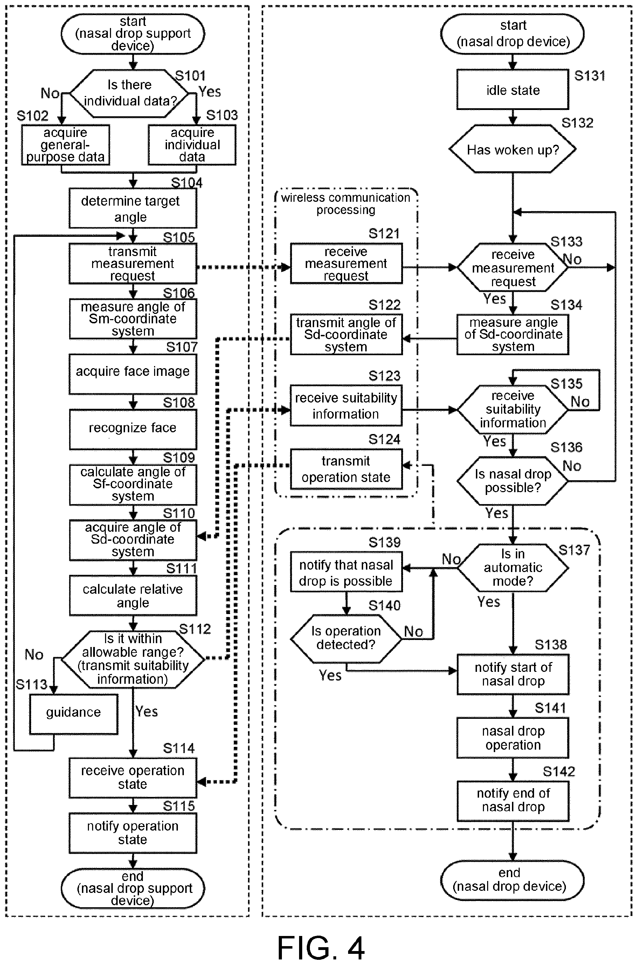

FIG. 4 is a flowchart showing an operation example of the nasal drop system 1 according to Embodiment 1. The broken line frame on the left side of the diagram mainly shows a processing flow executed by the processor 206 of the nasal drop support device 200. The broken line frame on the right side of the diagram mainly shows a processing flow executed by the processor 106 of the nasal drop device 100.

First, the processing flow of the nasal drop support device 200 is described. The processor 206 determines whether or not the individual data corresponding to the user of the nasal drop device 100 is in the memory 202 (S101). When the processor 206 determines that there is no individual data, the processor 206 acquires the general-purpose data from the memory 202 (S102), and when the processor 206 determines that there is individual data, the processor 206 acquires the individual data from the memory 202 (S103). Furthermore, a target value of the acquired general-purpose data or the individual data is determined as the target angle (S104).

Furthermore, the processor 206 transmits a measurement request for the tilt angle ($\alpha_d$, $\beta_d$, $\gamma_d$) of the Sd-coordinate system to the nasal drop device 100 via the wireless portion 208 (S105), and measures the tilt angle ($\alpha_m$, $\beta_m$, $\gamma_m$) of the Sm-coordinate system by the acceleration sensor 207 (S106). The tilt angle of the Sm-coordinate system is a tilt angle of the camera 210 with respect to the vertical direction, and is temporarily stored in the memory 202 as first angle information. Furthermore, the processor 206 acquires a face image of the user who puts the nasal drop device 100 in the nose from the camera 210 (S107). The face image is acquired, for example, in a manner that the processor 206 sets the nasal drop support device 200 to an imaging mode by the camera 210 and guides the user. The captured face image is, for example, an image as a moving image including a plurality of still images obtained by capturing images the face of the user from different angles. Furthermore, the processor 206 recognizes the face of the user from the face image (S108) and calculates the tilt angle ($\alpha_f$, $\beta_f$, $\gamma_f$) of the Sf-coordinate system from the recognized face (S109). For example, the processor 206 stereoscopically analyzes the face image including a plurality of still images and converts the face image into a three-dimensional image. The processor 206 detects the three-dimensional coordinates of the pupils of both eyes and the coordinates of the center position of the lips in the three-dimensional image. Furthermore, the processor 206 obtains a straight line connecting the midpoint of the line segment connecting the pupils of both eyes and the center of the lips as the reference line indicating the facial posture of the user. Because the coordinates of the three-dimensional image are in the Sm-coordinate system, the tilt angle of the reference line in the three-dimensional image is the tilt angle ($\alpha_f$, $\beta_f$, $\gamma_f$) of the face.

In addition, the processor 206 acquires the angle ($\alpha_d$, $\beta_d$, $\gamma_d$) of the Sd-coordinate system from the nasal drop device 100 via the wireless portion 208 in response to the measurement request in step S105 (S110). The angle ($\alpha_d$, $\beta_d$, $\gamma_d$) of the Sd-coordinate system is second angle information indicating the tilt angle of the nasal drop device 100 with respect to the vertical direction.

Furthermore, the processor 206 calculates the current relative angle between the reference line indicating the facial posture of the user and the reference line indicating the posture of the nasal drop device 100 (S111). More specifically, the processor 206 detects, as the first angle information, the tilt angle ($\alpha_m$, $\beta_m$, $\gamma_m$) of the camera 210 with respect to the vertical direction in step S106; acquires the second angle information indicating the tilt angle ($\alpha_d$, $\beta_d$, $\gamma_d$) of the nasal drop device 100 with respect to the vertical direction from the nasal drop device 100 in step S110; detects, as third angle information, the tilt angle ($\alpha_f$, $\beta_f$, $\gamma_f$) with respect to the image of the reference line indicating the facial posture of the user by analyzing the image captured by the camera 210 and the first angle information; and calculates the relative angle ($\alpha$, $\beta$, $\gamma$) based on the second angle information and the third angle information. More specifically, the processor 206 calculates, as the relative angle, the tilt angle ($\alpha$, $\beta$, $\gamma$) of the face coordinate system Sfd from the tilt angle ($\alpha_d$, $\beta_d$, $\gamma_d$) of the nasal drop device 100 in the Sd-coordinate system and the tilt angle ($\alpha_f$, $\beta_f$, $\gamma_f$) of the face in the Sf-coordinate system.

Furthermore, the processor 206 determines whether or not the calculated current relative angle is within the allowable range, that is, determines whether or not the deviation degree between the calculated relative angle and the target angle is within the allowable range, and transmits the determination result to the nasal drop device 100 as suitability information (S112). If the deviation degree is within the allowable range, it means that, for example, most of the droplets ejected from the nasal drop device 100 reach the olfactory part.

When the processor 206 determines that the relative angle is within the allowable range, the processor 206 receives an operation state from the nasal drop device 100 (S 1 14) and notifies the user of the operation state (S115).

On the other hand, when the processor 206 determines that the relative angle is not within the allowable range, the processor 206 notifies the user of the guidance indicating the determination result (S113), and the processing returns to step S 105.

Next, the processing flow of the nasal drop device 100 is described. The processor 106 of the nasal drop device 100 wakes up (S132) by a power-on operation in an idle state (S131).

Thereafter, the processor 106 determines whether or not the wireless portion 208 has received the measurement request from the nasal drop support device 200 to the acceleration sensor 107, that is, the measurement request of the tilt angle ($\alpha_d$, $\beta_d$, $\gamma_d$) of the Sd-coordinate system (S133). When the wireless portion 208 has received the measurement request (S121), the acceleration sensor 107 measures the tilt angle ($\alpha_d$, $\beta_d$, $\gamma_d$) of the Sd-coordinate system (S134), and transmits the measured tilt angle to the nasal drop device 100 (S122).

Furthermore, the processor 106 determines whether or not the wireless portion 208 has received the suitability information (S135). When the wireless portion 208 has received the suitability information (S123), the processor 106 determines whether or not the suitability information indicates nasal drop is possible, that is, whether or not the current relative angle is within the allowable range (S136). When the processor 106 determines that nasal drop is not possible, the processing returns to step S133.

When the processor 106 determines that nasal drop is possible, the processor 106 determines whether or not the nasal drop device 100 is in the automatic mode (S137). When the processor 106 determines that the nasal drop device 100 is in the automatic mode, the processor 106 notifies the user of the start of the nasal drop (S138), performs the nasal drop operation by the ejection portion 104 (S141), and notifies the user of the end of the nasal drop after the nasal drop operation is completed (S 142).

In addition, when the processor 106 determines that the nasal drop device 100 is not in the mode automatic, the processor 106 notifies the user that the nasal drop is possible (S139), waits for an operation instructing the user to start nasal drop (S140), and proceeds to step S 13 8 if the operation is performed.

In addition, in the processing of steps S137 to S142, the processor 106 transmits the operation state of the nasal drop device 100 to the nasal drop support device 200 via the wireless portion 108. The operation state indicates, for example, a state of the start of nasal drop, a state during the nasal drop operation, a state of the end of nasal drop, or the like. The nasal drop support device 200 notifies the user of the content of the operation state.

Moreover, in FIG. 4, when step S106 is referred to as first processing, steps S107 to S109 are referred to as second processing, and step S110 is referred to as third processing, the execution order of the first to third processing may not be this order. That is, the processor 206 may execute at least two of the first to third processing in parallel, or may execute the at least two processing in a different order. The parallel execution can be easily achieved when, for example, the processor 106 includes a plurality of processor cores.

As described above, according to the nasal drop device 100 of Embodiment 1, the following effects are achieved.

The nasal drop can be performed when the relative angle is appropriate, but not performed when the relative angle is not appropriate, and an appropriate amount of solution can be simply and easily administered to a specific site in the nasal cavity without waste.

In addition, because the suitability information is acquired from the other device via the wireless portion, an increase in the circuit scale and the circuit cost can be suppressed.

Furthermore, precision of the relative angle can be further improved, and the administration of an appropriate amount of solution to a specific site can be made simple and easy.

In addition, the amount of the ejected liquid can be controlled with a fine precision in the picolitre order, and the administration of an appropriate amount can be facilitated.

Furthermore, the appropriate use of the nasal drop device by the user can be supported. For example, if the suitability information indicates appropriateness, the operation of the user to start the nasal drop can be prompted, and if the suitability information indicates inappropriateness, the user can be prompted to adjust the angle of the nasal drop device with respect to the face of the user.

In addition, the nasal drop can be performed without missing the time during which the appropriate current relative angle is maintained, that is, before the current appropriate relative angle deviates.

According to the nasal drop support device 200 of Embodiment 1, an appropriate amount of nasal drop to a specific site can be supported without missing the time during which the appropriate current relative angle is maintained, that is, before the current appropriate relative angle deviates.

In addition, the nasal drop device can be controlled in a manner that the nasal drop is not performed when the above relative angle is not appropriate and the nasal drop is performed when the above relative angle is appropriate, and an appropriate amount of solution can be simply and easily administered to a specific site in the nasal cavity without waste.

Furthermore, precision of the relative angle can be further improved, and the administration of an appropriate amount of solution to a specific site can be made simple and easy.

In addition, an appropriate amount of solution can be simply and easily administered to a specific site in the nasal cavity of the user according to the individual data, and the same effect can also be achieved according to the general-purpose data.

Furthermore, the mobile device already owned by the user can be used as the nasal drop support device 200, and the cost burden on the user can be suppressed.

In addition, an appropriate amount of nasal drop can be easily achieved for a specific site without missing the time during which the appropriate current relative angle is maintained, that is, before the current appropriate relative angle deviates.

According to the nasal drop system 1 of Embodiment 1, by the notification, the nasal drop can be performed when the relative angle is appropriate but not performed when the relative angle is not appropriate, and an appropriate amount of solution can be simply and easily administered to a specific site in the nasal cavity without waste.

In addition, the relative angle can be calculated by analyzing the image, the acceleration sensor may not be included, and the increase in the circuit scale and the circuit cost can be suppressed.

Furthermore, precision of the relative angle can be further improved, and the administration of an appropriate amount of solution to a specific site can be made simple and easy.

In addition, the nasal drop support device can be configured based on a mobile device such as a smartphone or the like. In addition, the nasal drop support device can be configured based on a camera device having a wireless communication function.

Furthermore, precision of the relative angle can be further improved, and the administration of an appropriate amount of solution to a specific site can be made simple and easy.

(Embodiment 2, not according to the invention)

In Embodiment 2, a configuration example, which is not in accordance to the present invention, in which the acceleration sensors 107 and 207 of Embodiment 1 are not included is described.

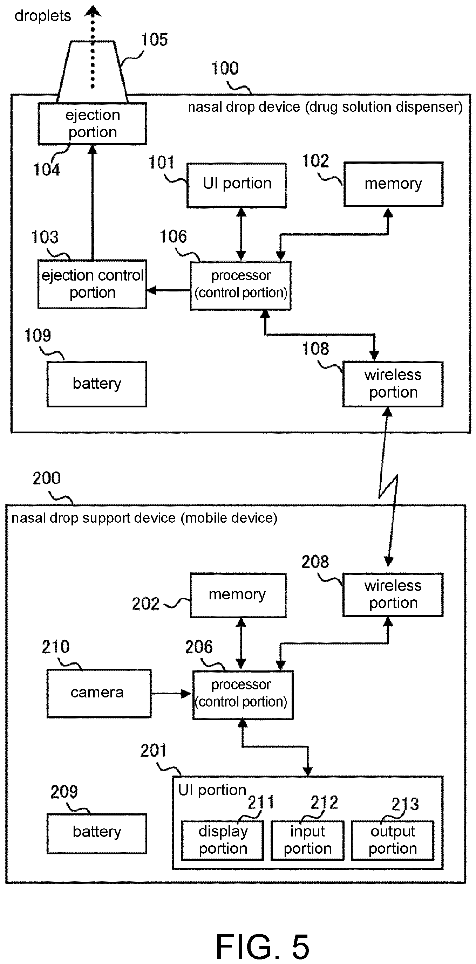

FIG. 5 is a block diagram showing a configuration example of the nasal drop system 1 according to Embodiment 2 (not according to the invention). Compared with FIG. 1A, the nasal drop system 1 in FIG. 5 is different in that the acceleration sensor 107 in the nasal drop device 100 has been deleted, the acceleration sensor 207 in the nasal drop support device 200 has been deleted, and the processing contents of the processors 106 and 206 are different. In the following, description is made avoiding repetition of description of the same points and focusing on the different points.

The processor 106 is different in not transmitting the tilt angle as the detection result of the acceleration sensor 107 to the nasal drop device 100 via the wireless portion 108.

The processor 206 cannot acquire the tilt angle from the acceleration sensors 107 and 207. Thus, with respect to the processor 206 in the embodiment, the following processing is added in which the image captured by the camera 210 is converted into a three-dimensional image and the current relative angle between the reference line indicating the facial posture of the user and the reference line indicating the posture of the nasal drop device 100 is calculated by analyzing the three-dimensional image.

Next, an operation example in the nasal drop system 1 of Embodiment 2 (not according to the invention) is described.

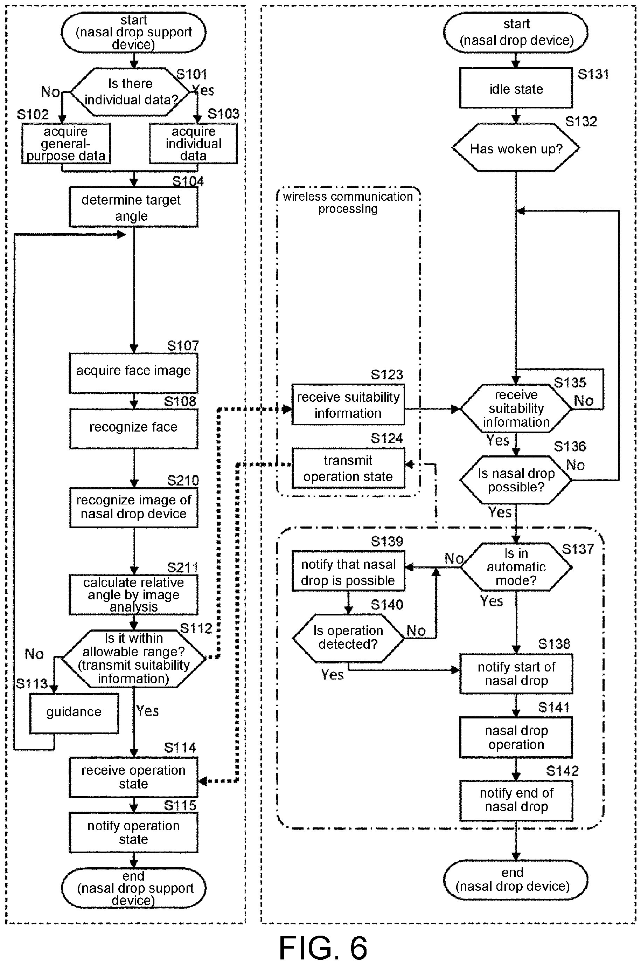

FIG. 6 is a flowchart showing an operation example of the nasal drop system according to Embodiment 2 (not according to the invention). Compared with FIG. 4, the flowchart in FIG. 6 is different in that steps S105, S106, S121, S122, S133 and S134 are deleted, and steps S210 and S211 are included instead of steps S109 to S111.

In step S108, the processor 206 stereoscopically analyzes a face image including a plurality of still images obtained from the camera 210, converts the face image into a three-dimensional image, and detects the reference line indicating the facial posture of the user.

In step S210, the processor 206 recognizes the nasal drop device 100 in the three-dimensional image and detects a ridge line of the nasal drop device 100. The processor 206 uses the ridge line as the reference line indicating the posture of the nasal drop device 100. Here, the reference line indicating the posture of the nasal drop device 100, which is detected from the three-dimensional image, belongs to the same mobile coordinate system Sm as the reference line indicating the facial posture of the user.

In step S211, the processor 206 calculates the relative angle between the reference line indicating the posture of the nasal drop device 100 and the reference line indicating the facial posture of the user.

Steps other than the above steps in FIG. 6 are substantially the same as those in FIG. 4.

Moreover, the "face image" in step S107 of FIG. 6 refers to an image including not only the face of the user who puts the nasal drop device 100 in the nose but also the nasal drop device 100.

In addition, in FIG. 6, the execution order of step S108 and step S210 may not be this order. After the completion of step S107, the processor 206 may execute step S108 and step S210 in parallel or in a different order. The parallel execution can be easily achieved when, for example, the processor 206 includes a plurality of processor cores.

In Embodiment 2 (not according to the invention), the relative angle is obtained by three-dimensionally analyzing the face image obtained from the camera 210 without using the acceleration sensors 107 and 207. The precision of the relative angle depends on the precision of the face image. For example, when the face image includes a plurality of still images captured from different angles, the processor 206 can analyze with high precision.

As described above, according to the nasal drop device 100 or the like of Embodiment 2 (not according to the invention), the relative angle can be calculated by analyzing the image, the acceleration sensor may not be included, and the increase in the circuit scale and the circuit cost can be suppressed.

(Embodiment 3)

In Embodiment 3, described is a configuration example of the nasal drop system 1 including the nasal drop support device 200 configured based on the hardware of a camera device having a wireless function.

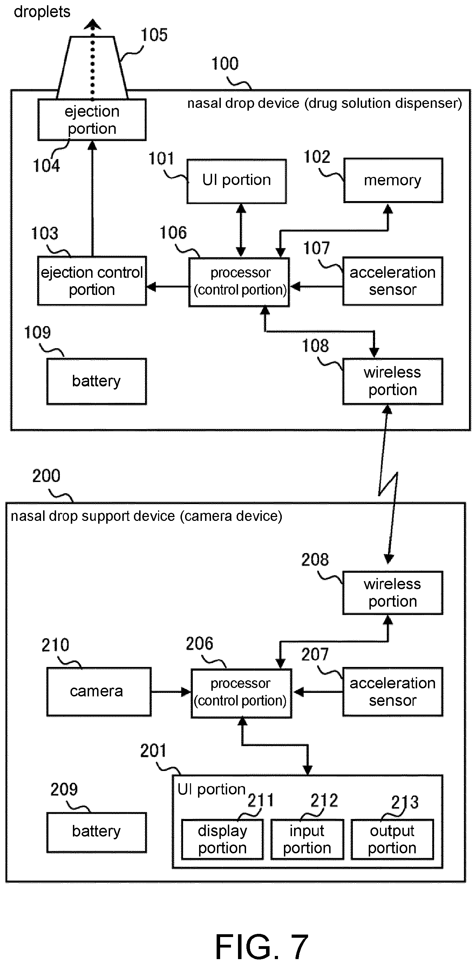

FIG. 7 is a block diagram showing a configuration example of the nasal drop system 1 according to Embodiment 3. Compared with FIG. 1A, the nasal drop system 1 in FIG. 7 is different in that the nasal drop support device 200 is configured based on the hardware of the camera device having a wireless function, and that the calculation of the relative angle and the generation of the suitability information are performed by the nasal drop device 100 instead of the nasal drop support device 200. In the following, description is made avoiding repetition of description of the same points and focusing on the different points.

The nasal drop support device 200 is configured based on the hardware of the camera device having a wireless function. Therefore, the processor 206 is used for image processing and does not perform processing such as the calculation of the relative angle or the like.

The nasal drop device 100 executes processing such as the calculation of the relative angle executed by the processor 206 in Embodiment 1.

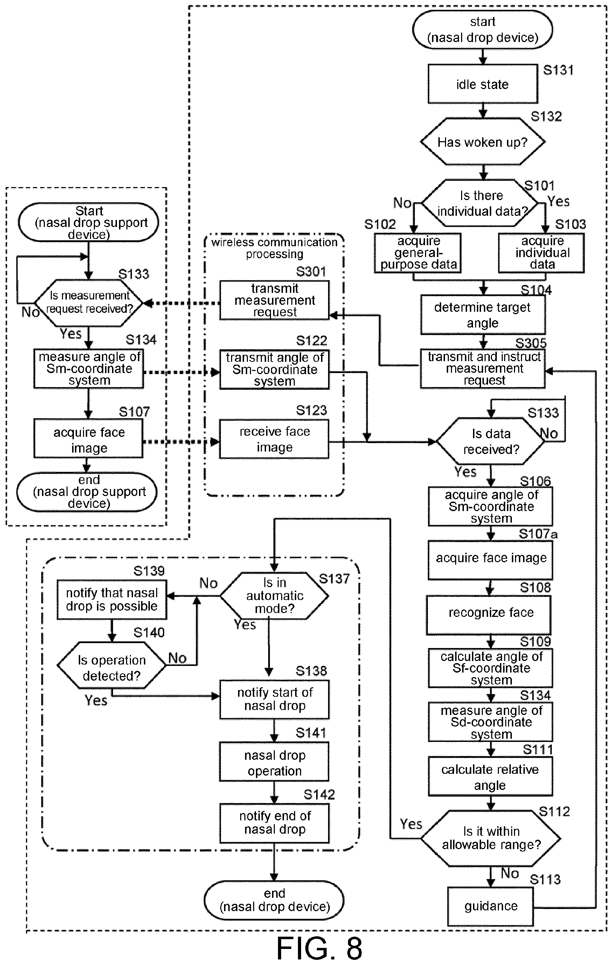

FIG. 8 is a flowchart showing an operation example of the nasal drop system 1 according to Embodiment 3. Compared with FIG. 4, the flowchart in FIG. 8 is different in that a step sharing ratio between the nasal drop device 100 and the nasal drop support device 200 is significantly different. That is, steps other than the steps S133, S134, and S107 performed by the nasal drop support device 200 are changed to be executed by the nasal drop device 100. In addition, step S301 is added. In order to avoid description repetition, the same step numbers are assigned to the steps having the same processing contents in FIGS. 8 and 6.

Moreover, in FIG. 8, the execution order of step S134 and step S107 may not be this order. For example, the processor 206 may execute step S134 and step S107 in parallel or in a different order. The parallel execution can be easily achieved when, for example, the processor 206 includes a plurality of processor cores.

As described above, because it is sufficient that the nasal drop support device 200 of the nasal drop system 1 according to Embodiment 3 has a function of transmitting the captured face image to the nasal drop device 100, the nasal drop support device 200 may be configured based on a camera having a wireless function. The nasal drop support device 200 can be based on, for example, a camera device such as a monitoring camera, a Web camera or the like.

(Embodiment 4, not according to the invention)

In Embodiment 4, a configuration example (not according to the invention) in which the acceleration sensors 107 and 207 of Embodiment 3 are not included is described.

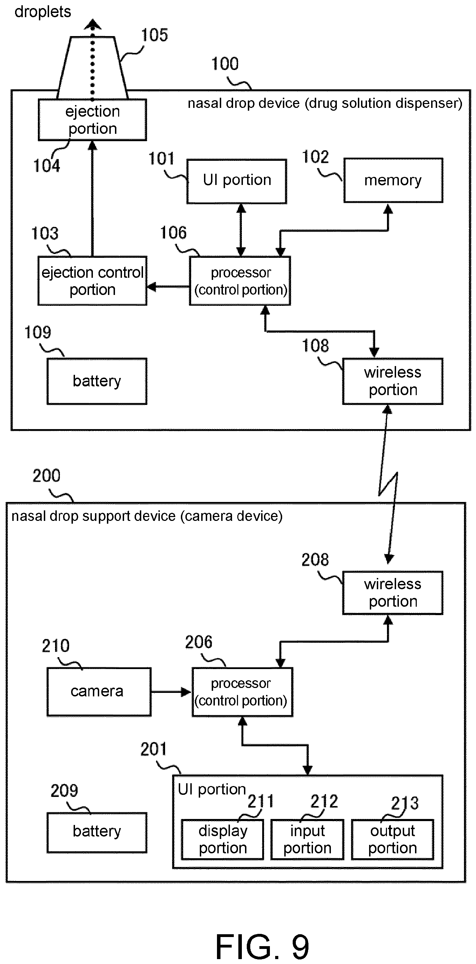

FIG. 9 is a block diagram showing the configuration example of the nasal drop system 1 according to Embodiment 4 (not according to the invention). Compared with FIG. 7, the nasal drop system 1 in FIG. 9 is different in that the acceleration sensor 107 in the nasal drop device 100 is deleted, the acceleration sensor 207 in the nasal drop support device 200 is deleted, and processing contents of the processors 106 and 206 are different. In the following, description is made avoiding repetition of description of the same points and focusing on different points.

The processor 106 is different in not transmitting the tilt angle as the detection result of the acceleration sensor 107 to the nasal drop device 100 via the wireless portion 108.

The processor 206 cannot acquire the tilt angle from the acceleration sensors 107 and 207. Thus, with respect to the processor 206 in the embodiment, the following processing is added in which the image captured by the camera 210 is converted into a three-dimensional image and the current relative angle between the reference line indicating the facial posture of the user and the reference line indicating the posture of the nasal drop device 100 is calculated by analyzing the three-dimensional image.

Next, an operation example in the nasal drop system 1 of Embodiment 4 (not according to the invention) is described.

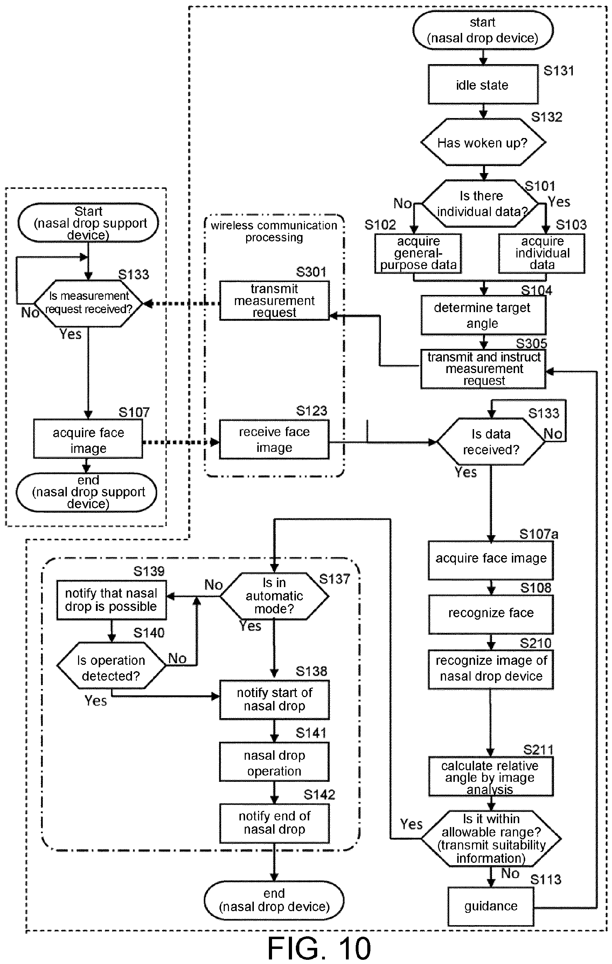

FIG. 10 is a flowchart showing the operation example of the nasal drop system according to Embodiment 4 (not according to the invention). Compared with FIG. 8, the flowchart of FIG. 10 is changed in a manner that the nasal drop device 100 shares steps other than steps 133 and 107 processed by the nasal drop support device 200. In order to avoid description repetition, the same step numbers are assigned to the steps having the same processing contents in FIG. 10 and FIG. 8.

Moreover, in step S107 of FIG. 10, the face image is captured by the camera 210, and in step S 107a, this face image is acquired by wireless communication. The face image here refers to an image including not only the face of the user who puts the nasal drop device 100 in the nose but also the nasal drop device 100.

In addition, in FIG. 10, the execution order of step S108 and step S210 may not be this order. After the completion of step S107a, the processor 106 may execute step S108 and step S210 in parallel or in a different order. The parallel execution can be easily achieved when, for example, the processor 106 includes a plurality of processor cores.

As described above, according to the nasal drop device 100 or the like of Embodiment 4 (not according to the invention), the relative angle can be calculated by analyzing the image, the acceleration sensor may not be included, and the increase in the circuit scale and the circuit cost can be suppressed.

(Embodiment 5)

A configuration example is described in which the nasal drop device 100 of Embodiment 5 has functions of both the nasal drop device 100 and the nasal drop support device 200 of Embodiment 1.

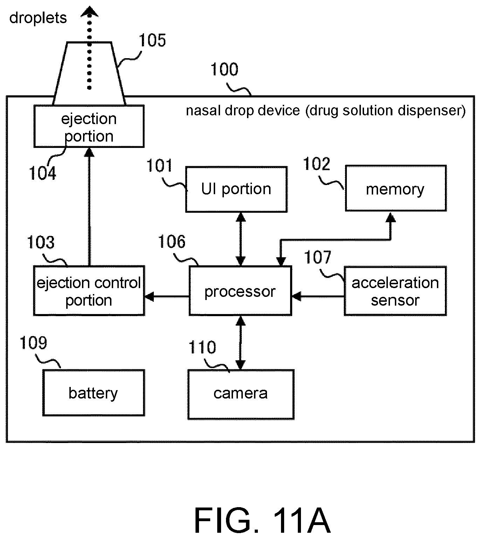

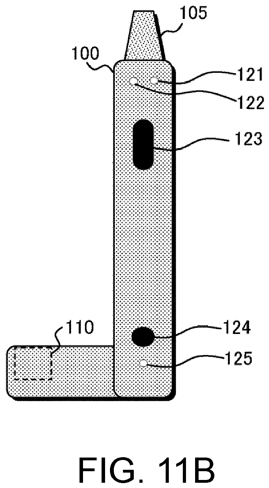

FIG. 11A is a block diagram showing the configuration example of the nasal drop device according to Embodiment 5. In addition, FIG. 11B is a block diagram showing an appearance example of the nasal drop device according to Embodiment 5.

Compared with the nasal drop device 100 in FIG. 1A, the nasal drop device 100 in FIG. 11A is different in that a camera 110 is added and the processor 106 also executes the processing of the processor 206. Compared with the nasal drop device 100 in FIG. 1B, the nasal drop device 100 in FIG. 11B is different in that an arm with the camera 110 is added below the main body of the nasal drop device 100. Description is made below focusing on the different points.

The arm with the camera 110 may be housed in, for example, the main body of the nasal drop device 100, or may be removable.

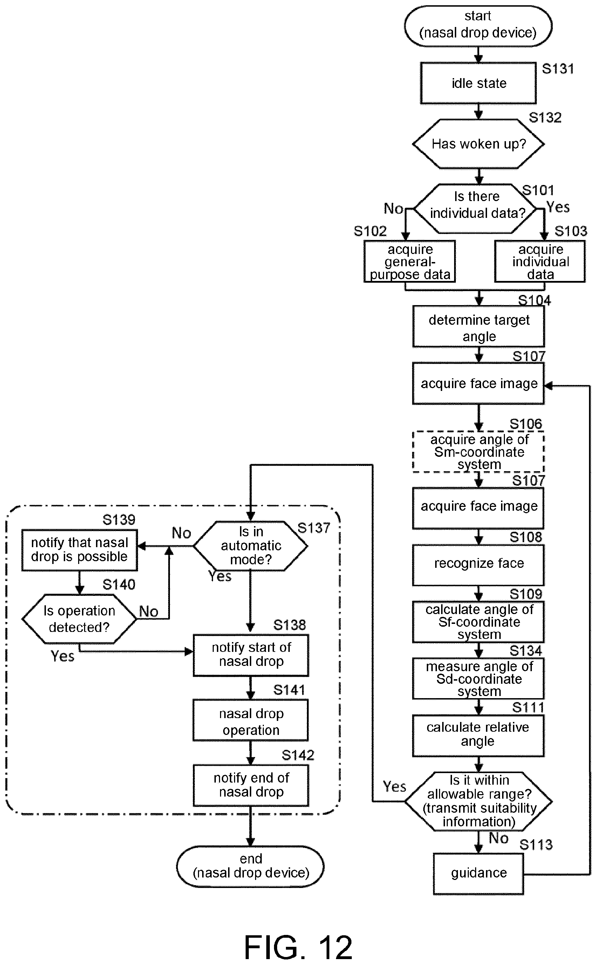

FIG. 12 is a flowchart showing an operation example of the nasal drop device according to Embodiment 5. FIG. 12 is different from FIG. 8 in that steps S301 and S123 are deleted and the processor 106 executes step S107 that is performed by the processor 206 in FIG. 10. The same step numbers are assigned to the steps having the same processing contents in FIG. 12 and FIG. 8.

Moreover, the processor 106 may execute one of steps S106 and S134 in FIG. 12 and the other step may be omitted. The reason thereof is that, in Embodiment 5, the mobile coordinate system Sm and the dispenser coordinate system Sd are the same coordinate system, and thus the tilt angle ($\alpha_m$, $\beta_m$, $\gamma_m$) of the mobile coordinate system Sm and the tilt angle ($\alpha_d$, $\beta_d$, $\gamma_d$) of the dispenser coordinate system Sd are the same. Therefore, the processor 106 does not need to handle the mobile coordinate system Sm in the first place in Embodiment 5. Alternatively, the processor 106 can use the processing of Embodiments 1 to 4 by regarding the two coordinate systems, that is, the mobile coordinate system Sm and the dispenser coordinate system Sd as the same coordinate system.

In addition, in FIG. 12, the execution order of the processing of steps S107 to S109 and the processing of step S134 may not be this order. The processor 106 may execute the processing of steps S107 to S109 and the processing of step S134 in parallel or in a reverse order. The parallel execution can be easily achieved when, for example, the processor 206 includes a plurality of processor cores.

As described above, according to the nasal drop device 100 of Embodiment 5, the nasal drop device 100 alone can achieve the same effect as that of Embodiments 1 to 4.

(Embodiment 6, not according to the invention)

In Embodiment 6, a configuration example (not according to the invention) in which the acceleration sensor 107 of Embodiment 5 is not included is described.

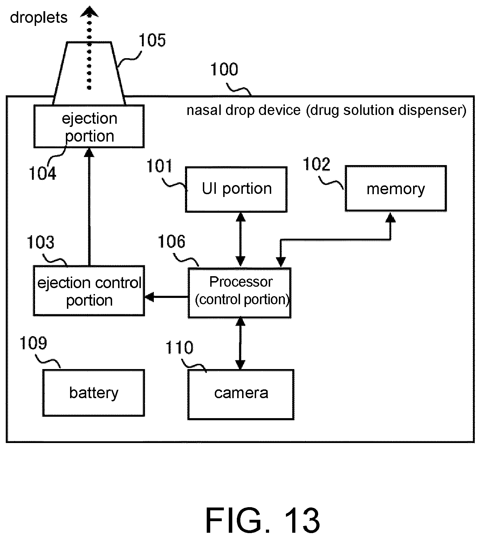

FIG. 13 is a block diagram showing a configuration example of the nasal drop device 100 according to Embodiment 6 (not according to the invention). Compared with FIG. 12, the nasal drop device 100 in FIG. 13 is different in that the acceleration sensor 107 in the nasal drop device 100 is deleted and the processing content of the processor 106 is different. Next, an operation example in the nasal drop system 1 of Embodiment 6 (not according to the invention) is described.

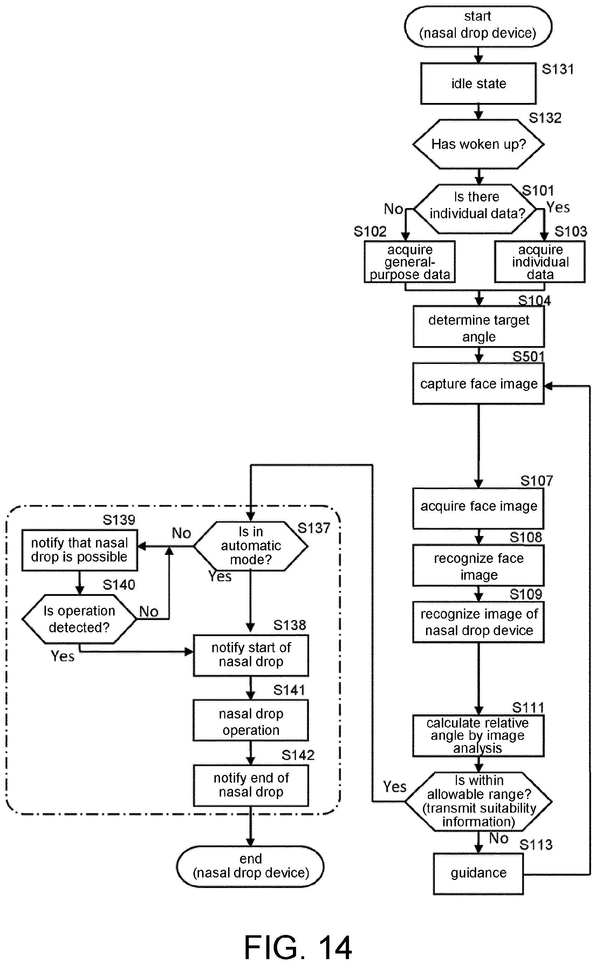

FIG. 14 is a flowchart showing an operation example of the nasal drop system according to Embodiment 6 (not according to the invention). Compared with FIG. 10, the flowchart of FIG. 14 is different in that steps S301 and S123 are deleted and the processor 106 executes step S107 that is performed by the processor 206 in FIG. 10. The same step numbers are assigned to the steps having the same processing contents in FIG. 12 and FIG. 10.

Moreover, the "face image" in step S501 of FIG. 14 refers to an image including not only the face of the user who puts the nasal drop device 100 in the nose but also the nasal drop device 100.

In addition, in FIG. 14, the execution order of step S108 and step S109 may not be this order. After the completion of step S107, the processor 106 may execute step S108 and step S109 in parallel or in a different order. The parallel execution can be easily achieved when, for example, the processor 106 includes a plurality of processor cores.

As described above, according to the nasal drop device 100 of Embodiment 6 (not according to the invention), the nasal drop device 100 alone can calculate the relative angle by analyzing the image, the acceleration sensor may not be included, and the increase in the circuit scale and the circuit cost can be suppressed.

(Embodiment 7)

In Embodiment 7, a configuration example of the nasal drop system 1 capable of further connecting a server device and a network router for Embodiments 1 to 4 is described.

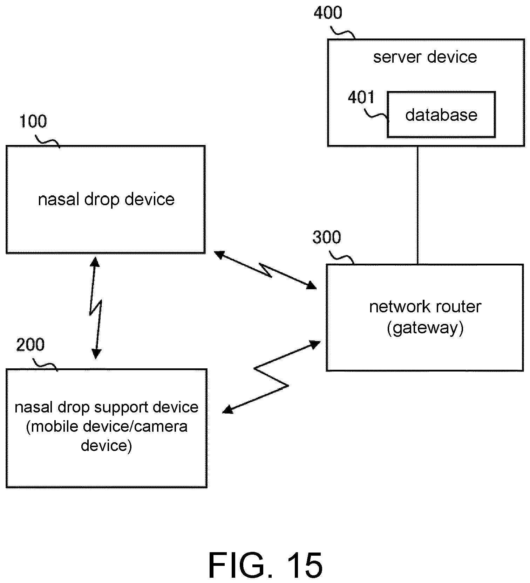

FIG. 15 is a block diagram showing the configuration example of the nasal drop system according to Embodiment 7. The nasal drop system 1 in the diagram includes the nasal drop device 100, the nasal drop support device 200, a network router 300, and a server device 400.

The nasal drop device 100 and the nasal drop support device 200 may be the same as the nasal drop device 100 and the nasal drop support device 200 according to any one of the Embodiments 1 to 4.

The network router 300 relays a communication message between the nasal drop device 100 and the nasal drop support device 200, and the server device 400.

The server device 400 receives the face image, information indicating the tilt angle, and information indicating the relative angle from the nasal drop support device 200 or the nasal drop device 100, and accumulates the information in a database 401. Statistical processing is performed on the accumulated data, and individual data and general-purpose data are corrected to be more accurate.

Moreover, the server device 400 may calculate the relative angle and generate suitability information as necessary.

(Embodiment 8)

In Embodiment 8, as a variation example of Embodiment 7, a configuration example of the nasal drop system 1 that can be connected to the server device 400 via a wireless base station for smartphones is described.

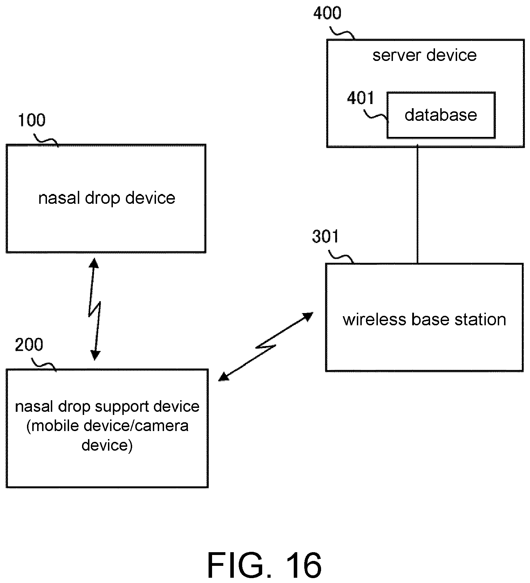

FIG. 16 is a block diagram showing the configuration example of the nasal drop system according to Embodiment 8. Compared with FIG. 15, FIG. 16 is different in having a wireless base station 301 instead of the network router 300. Due to this difference, the nasal drop support device 200 can be connected to the server device 400 via the wireless base station 301. The operation of the nasal drop system 1 according to Embodiment 8 is the same as that of Embodiment 7.

As described above, the nasal drop device according to Embodiments 1 to 8 includes: an ejection portion that ejects droplets into the nasal cavity; and a control portion that acquires suitability information indicating whether or not a current relative angle between a reference line indicating the facial posture of the user who puts the nasal drop device in the nose and a reference line indicating the posture of the nasal drop device is suitable for the nasal drop, and controls the ejection operation of the ejection portion according to the suitability information.

Accordingly, the nasal drop can be performed when the relative angle is appropriate, but not performed when the relative angle is not appropriate, and an appropriate amount of solution can be simply and easily administered to a specific site in the nasal cavity without waste.

For example, the nasal drop device may further include a wireless portion for wireless communication. The control portion may acquire the suitability information from another device via the wireless portion.

Accordingly, because the suitability information is acquired from another device via the wireless portion, an increase in the circuit scale and the circuit cost can be suppressed.

For example, the nasal drop device may further include an acceleration sensor that detects a tilt angle of the nasal drop device with respect to a vertical direction. The wireless portion may transmit the tilt angle detected by the acceleration sensor to the another device.

Accordingly, precision of the relative angle can be further improved, and the administration of an appropriate amount of solution to a specific site can be made simple and easy.

For example, the nasal drop device may further include a camera that captures an image of the face of the user who puts the nasal drop device in the nose. The control portion may calculate the relative angle by analyzing the image captured by the camera, determine whether or not the deviation degree between the relative angle and the target angle is within the allowable range, and acquire the determination result as the suitability information.

Accordingly, the relative angle can be calculated by analyzing the image, the acceleration sensor may not be included, and the increase in the circuit scale and the circuit cost can be suppressed.

For example, the nasal drop device may further include an acceleration sensor that detects a tilt angle of the nasal drop device with respect to a vertical direction. The control portion may calculate the relative angle by analyzing the image captured by the camera based on the tilt angle.

Accordingly, precision of the relative angle can be further improved, and the administration of an appropriate amount of solution to a specific site can be made simple and easy.

For example, in the nasal drop device, the ejection portion may eject droplets of several picolitres to several tens of picolitres in a piezoelectric way or a thermal way.

Accordingly, the amount of the ejected liquid can be controlled with a fine precision in the picolitre order, and the administration of an appropriate amount can be facilitated.

For example, the nasal drop device may further include a notification portion that notifies the user of the content of the suitability information by using at least one of light, sound, and vibration.

Accordingly, the appropriate use of the nasal drop device by the user can be supported. For example, if the suitability information indicates appropriateness, the operation of the user to start the nasal drop can be prompted, and if the suitability information indicates inappropriateness, the user can be prompted to adjust the angle of the nasal drop device with respect to the face of the user.

For example, in the control device, when the suitability information indicates appropriateness, the control portion may cause the ejection portion to start the ejection operation without waiting for the operation instructing start of the nasal drop from the user.

Accordingly, the nasal drop can be performed without missing the time during which the appropriate current relative angle is maintained, that is, before the current appropriate relative angle deviates.

In addition, the nasal drop support device according to Embodiments 1 to 8 is a nasal drop support device that supports the use of a nasal drop device by a user and includes: a camera that captures an image of the face of the user who puts the nasal drop device in the nose; a processor that calculates, based on the image captured by the camera, a current relative angle between a reference line indicating the facial posture of the user and a reference line indicating the posture of the nasal drop device, and generates suitability information indicating whether or not the deviation degree between the relative angle and the target angle is within the allowable range; and a notification portion that notifies the user of the content of the suitability information.

Accordingly, an appropriate amount of nasal drop to a specific site can be supported without missing the time during which the appropriate current relative angle is maintained, that is, before the current appropriate relative angle deviates.

The nasal drop support device further includes a communication portion that transmits the suitability information to the nasal drop device.

Accordingly, the nasal drop device can be controlled in a manner that the nasal drop is not performed when the above relative angle is not appropriate and the nasal drop is performed when the above relative angle is appropriate, and an appropriate amount of solution can be simply and easily administered to a specific site in the nasal cavity without waste.

The nasal drop support device further includes an acceleration sensor that detects a tilt angle of the camera with respect to a vertical direction as first angle information. The communication portion receives, from the nasal drop device, second angle information indicating a tilt angle of the nasal drop device with respect to the vertical direction. The processor detects, as third angle information, a tilt angle between the reference line indicating the facial posture of the user and the reference line indicating the posture of the nasal drop support device, by analyzing the image captured by the camera and the first angle information, and may calculate the relative angle based on the second angle information and the third angle information.

Accordingly, precision of the relative angle can be further improved, and the administration of an appropriate amount of solution to a specific site can be made simple and easy.

For example, the nasal drop support device may further include a memory that stores in advance individual data indicating, as a target, a relative angle between a reference line indicating the facial posture of each user and the reference line of the nasal drop device, and general-purpose data indicating, as an average target, the relative angle between reference lines indicating facial postures of a plurality of users and the reference line indicating the posture of the nasal drop device. The processor may select one of the individual data and the general-purpose data, and determine the target angle from the selection result.

Accordingly, an appropriate amount of solution can be simply and easily administered to a specific site in the nasal cavity of the user according to the individual data without waste, and the same effect can also be achieved according to the general-purpose data.

For example, the nasal drop support device may be a mobile device.

Accordingly, the mobile device already owned by the user can be used as the nasal drop support device 200, and the cost burden on the user can be suppressed.

The nasal drop support device 200 further includes a notification portion that notifies the user of the content of the suitability information by using at least one of light, sound, and vibration.

Accordingly, an appropriate amount of nasal drop can be easily achieved for a specific site without missing the time during which the appropriate current relative angle is maintained, that is, before the current appropriate relative angle deviates.

In addition, the nasal drop system according to Embodiments 1 to 8 includes: a nasal drop device that ejects droplets into the nasal cavity; and a nasal drop support device that supports use of the nasal drop device by a user. One of the nasal drop support device and the nasal drop device has an acquisition portion that acquires an image obtained by capturing an image of the face of the user who puts the nasal drop device in the nose. One of the nasal drop support device and the nasal drop device has a processor that calculates, based on the image, a current relative angle between a reference line indicating the facial posture of the user and a reference line indicating the posture of the nasal drop device, and generates suitability information indicating whether or not the deviation degree between the relative angle and the target angle is within the allowable range. One of the nasal drop support device and the nasal drop device has a notification portion that notifies the user of the content of the suitability information.

Accordingly, by the notification, the nasal drop can be performed when the relative angle is appropriate, but not performed when the relative angle is not appropriate, and an appropriate amount of solution can be simply and easily administered to a specific site in the nasal cavity without waste.

For example, one of the nasal drop support device and the nasal drop device may have a camera that generates an image obtained by capturing an image of the face of the user who puts the nasal drop device in the nose. The acquisition portion may acquire the image from the camera. The processor may recognize the face of the user and the nasal drop device from the image, specify the reference line indicating the facial posture of the user and the reference line indicating the posture of the nasal drop device from the recognition result, and calculate the relative angle from the specification result.

Accordingly, the relative angle can be calculated by analyzing the image, the acceleration sensor may not be included, and the increase in the circuit scale and the circuit cost can be suppressed.

For example, at least one of the nasal drop support device and the nasal drop device may have an acceleration sensor that detects a tilt angle with respect to a vertical direction, and the processor may calculate the relative angle by using the tilt angle detected by the acceleration sensor.

Accordingly, precision of the relative angle can be further improved, and the administration of an appropriate amount of solution to a specific site can be made simple and easy.

For example, the nasal drop support device may include the acquisition portion, the processor, and the camera.

Accordingly, the nasal drop support device can be configured based on a mobile device such as a smartphone or the like. In addition, the nasal drop support device can be configured based on a camera device having a wireless communication function.

For example, the nasal drop device may include a first acceleration sensor that serves as the acceleration sensor, and the nasal drop support device may include the acquisition portion, the processor, the camera, and a second acceleration sensor that serves as the acceleration sensor.

Accordingly, precision of the relative angle can be further improved, and the administration of an appropriate amount of solution to a specific site can be made simple and easy.

The nasal drop administration method according to Embodiments 1 to 8 is a nasal drop administration method of a nasal drop device that ejects droplets into the nasal cavity, in which suitability information is acquired that indicates whether or not a current relative angle between a reference line indicating the facial posture of the user who puts the nasal drop device in the nose and a reference line indicating the posture of the nasal drop device is suitable for the nasal drop; and the ejection operation is controlled according to the suitability information.

Accordingly, the nasal drop can be performed when the relative angle is appropriate, but not performed when the relative angle is not appropriate, an appropriate amount of solution can be simply and easily administered to a specific site in the nasal cavity without waste.

The nasal drop support method according to Embodiments 1 to 8 is a support method of a nasal drop support device that supports the use of a nasal drop device by a user, in which an image of the face of the user who puts the nasal drop device in the nose is captured by a camera; a current relative angle between a reference line indicating the facial posture of the user and a reference line indicating the posture of the nasal drop device is calculated based on the image captured by the camera; suitability information that indicates whether or not the deviation degree between the relative angle and the target angle is within the allowable range is generated; and the content of the suitability information is notified to the user.

Accordingly, an appropriate amount of nasal drop to a specific site can be supported without missing the time during which the appropriate current relative angle is maintained, that is, before the current appropriate relative angle deviates.

The operation method of a nasal drop system according to Embodiments 1 to 8 is an operation method of a nasal drop system including a nasal drop device that ejects droplets into the nasal cavity and a nasal drop support device that supports the use of the nasal drop device by a user, in which one of the nasal drop support device and the nasal drop device acquires an image obtained by capturing an image of the face of the user who puts the nasal drop device in the nose; one of the nasal drop support device and the nasal drop device calculates, based on the image, a current relative angle between a reference line indicating the facial posture of the user and a reference line indicating the posture of the nasal drop device; one of the nasal drop support device and the nasal drop device generates suitability information indicating whether or not the deviation degree between the relative angle and the target angle is within the allowable range; and one of the nasal drop support device and the nasal drop device notifies the user of the content of the suitability information.

Accordingly, by the notification, the nasal drop can be performed when the relative angle is appropriate, but not performed when the relative angle is not appropriate, and an appropriate amount of solution can be simply and easily administered to a specific site in the nasal cavity without waste.

The program of a nasal drop device according to Embodiments 1 to 8 is executed in a computer included in a nasal drop device that ejects droplets into the nasal cavity, and is executed to: acquire suitability information that indicates whether or not a current relative angle between a reference line indicating the facial posture of a user who puts the nasal drop device in the nose and a reference line indicating the posture of the nasal drop device is suitable for the nasal drop; and control the ejection operation according to the suitability information.

Accordingly, the nasal drop can be performed when the relative angle is appropriate, but not performed when the relative angle is not appropriate, and an appropriate amount of solution can be simply and easily administered to a specific site in the nasal cavity without waste.

The program of a nasal drop support device according to Embodiments 1 to 8 is executed in a computer included in a nasal drop support device that supports the use of a nasal drop device by a user, and is executed to: capture an image of the face of the user who puts the nasal drop device in the nose by a camera; calculate, based on the image captured by the camera, a current relative angle between a reference line indicating the facial posture of the user and a reference line indicating the posture of the nasal drop device; generate suitability information indicating whether or not the deviation degree between the relative angle and the target angle is within the allowable range; and notify the user of the content of the suitability information.

Accordingly, an appropriate amount of nasal drop to a specific site can be supported without missing the time during which the appropriate current relative angle is maintained, that is, before the current appropriate relative angle deviates.

The program of a nasal drop system according to Embodiments 1 to 8 is executed in a computer included in a nasal drop system including a nasal drop device that ejects droplets into the nasal cavity and a nasal drop support device that supports the use of the nasal drop device by a user, and the program is executed in order that: one of the nasal drop support device and the nasal drop device acquires an image obtained by capturing an image of the face of the user who puts the nasal drop device in the nose; one of the nasal drop support device and the nasal drop device calculates, based on the image, a current relative angle between a reference line indicating the facial posture of the user and a reference line indicating the posture of the nasal drop device; one of the nasal drop support device and the nasal drop device generates suitability information indicating whether or not the deviation degree between the relative angle and the target angle is within the allowable range; and one of the nasal drop support device and the nasal drop device notifies the user of the content of the suitability information.

Accordingly, by the notification, the nasal drop can be performed when the relative angle is appropriate, but not performed when the relative angle is not appropriate, and an appropriate amount of solution can be simply and easily administered to a specific site in the nasal cavity without waste.

Although the nasal drop device, the nasal drop support device and the like according to the embodiments of the disclosure have been described above, the disclosure is not limited to these embodiments.

In addition, specifically, each of the above devices may be configured as a computer system including a microprocessor, a ROM, a RAM, a hard disk drive, a display unit, a keyboard, a mouse, and the like. The computer program is stored in the RAM or the hard disk drive. Each device achieves the function thereof by operating the microprocessor according to the computer program. Here, the computer program is configured by combining a plurality of instruction codes indicating commands to a computer in order to achieve a predetermined function.

Furthermore, a part or all of the components configuring each of the above devices may be configured by one system large scale integration (LSI). The system LSI is an ultra-multifunctional LSI manufactured by integrating a plurality of components on one chip, and includes a computer system including, for example, a microprocessor, a ROM, a RAM, and the like. In this case, the computer program is stored in the ROM. The system LSI achieves the function thereof by operating the microprocessor according to the computer program.

Furthermore, a part or all of the components configuring each of the above devices may be configured by an IC card or a single module that can be attached to and detached from each device. The IC card or the module is a computer system configured by a microprocessor, a ROM, a RAM, and the like. The IC card or the module may include the above-mentioned ultra-multifunctional LSI. The IC card or the module achieves the function thereof by operating the microprocessor according to a computer program. The IC card or the module may have tamper resistance.

In addition, the disclosure may be the methods shown above. In addition, the disclosure may be the computer programs that realize these methods by a computer, or may be a digital signal composed of the above computer programs.

Furthermore, the disclosure may be obtained by recording the computer program or the digital signal in a computer-readable non-temporary recording medium, such as a flexible disc, a hard disk, a CD-ROM, a MO, a DVD, a DVD-ROM, a DVD-RAM, a Blu-ray (registered trademark) Disc (BD), a semiconductor memory, or the like. In addition, the disclosure may be the digital signal recorded on these non-temporary recording media.

In addition, the disclosure may transmit the computer program or the digital signal via a telecommunication line, a wireless or wired communication line, a network typified by the Internet, data broadcasting, or the like.

In addition, the disclosure may be a computer system including a microprocessor and a memory. The memory may store the computer program, and the microprocessor may operate according to the computer program.

In addition, by recording and transferring the program or the digital signal onto the non-temporary recording medium, or by transferring the program or the digital signal via the network or the like, the disclosure may be carried out by another independent computer system.

Furthermore, the above embodiments and the above variation examples may be combined respectively.

[Industrial applicability]

The disclosure can be used for a nasal drop device that ejects droplets into the nasal cavity, a nasal drop support device that supports use of a nasal drop device by a user, and the like.

[Reference Signs List]

1: nasal drop system; 100: nasal drop device; 101, 201: UI portion; 102, 202: memory; 103: ejection control portion; 104: ejection portion; 105: nose piece; 106, 206: processor; 107, 207: acceleration sensor; 108, 208: wireless portion; 109, 209: battery; 110, 210: camera; 121, 122: LED; 123: operation button; 124: power button; 125: power LED; 200: nasal drop support device; 211: display portion; 212: input portion; 213: output portion; 300: network router; 301: wireless base station; 400: server device; 401: database; R1: olfactory region.

**Claims**

1. A nasal drop support device (200) configured to support use of a nasal drop device (100) by a user, comprising:

   a camera (210) configured to capture an image of the face of the user who puts the nasal drop device (100) in the nose;
   a processor (206) configured to calculate, based on the image captured by the camera (210), a current relative angle between a reference line indicating the facial posture of the user and a reference line indicating the posture of the nasal drop device (100), and generate suitability information indicating whether or not the deviation degree between the relative angle and a target angle is within an allowable range;
   a notification portion configured to notify the user of the content of the suitability information;
   a communication portion configured to transmit the suitability information to the nasal drop device (100); and
   an acceleration sensor (207) configured to detect a tilt angle of the camera (210) with respect to a vertical direction as first angle information, wherein
   the communication portion is configured to receive, from the nasal drop device (100), second angle information indicating a tilt angle of the nasal drop device (100) with respect to the vertical direction; the nasal drop support device (200) being **characterized in that**
   the processor (206) is configured to detect, as third angle information, a tilt angle between the reference line indicating the facial posture of the user and a reference line indicating the posture of the nasal drop support device (200), by analyzing the image captured by the camera (210) and the first angle information, and calculate the relative angle based on the second angle information and the third angle information.

2. The nasal drop support device (200) according to claim 1, further comprising

   a memory (202) configured to store in advance individual data indicating, as a target, a relative angle between a reference line indicating the facial posture of each user and the reference line of the nasal drop device (100), and general-purpose data indicating, as an average target, a relative angle between reference lines indicating facial postures of a plurality of users and the reference line indicating the posture of the nasal drop device (100), wherein

the processor (206) is configured to select one of the individual data and the general-purpose data, and determine the target angle from the selection result.

3. The nasal drop support device (200) according to any one of claims 1 to 2, wherein
the nasal drop support device (200) is a mobile device.

4. The nasal drop support device (200) according to any one of claims 1 to 3, wherein the notification portion is configured to notify the user of the content of the suitability information by using at least one of light, sound, and vibration.

5. A nasal drop system (1), comprising:

a nasal drop device (100) configured to eject droplets into the nasal cavity; and
the nasal drop support device (200) according to claim 1, wherein
one of the nasal drop support device (200) and the nasal drop device (100) has an acquisition portion configured to acquire an image obtained by capturing an image of the face of the user who puts the nasal drop device (100) in the nose.

6. The nasal drop system (1) according to claim 5, wherein

the nasal drop support device (200) comprises the acquisition portion, the processor (206), and the camera (210), and
the acquisition portion is configured to acquire the image from the camera (210).

7. A support method of a nasal drop support device (200) that supports use of a nasal drop device (100) by a user, comprising:

capturing an image of the face of the user who puts the nasal drop device (100) in the nose by a camera (210); and
calculating, based on the image captured by the camera (210), a current relative angle between a reference line indicating the facial posture of the user and a reference line indicating the posture of the nasal drop device (100), and generating suitability information indicating whether or not the deviation degree between the relative angle and a target angle is within an allowable range,
notifying the user of the content of the suitability information;
transmitting the suitability information to the nasal drop device (100);
detecting a tilt angle of the camera (210) with respect to a vertical direction as first angle information;
receiving, from the nasal drop device (100), second angle information indicating a tilt angle of the nasal drop device (100) with respect to the vertical direction; and
the support method being **characterized in** further comprising detecting, as third angle information, a tilt angle between the reference line indicating the facial posture of the user and a reference line indicating the posture of the nasal drop support device (200), by analyzing the image captured by the camera (210) and the first angle information, and calculating the relative angle based on the second angle information and the third angle information.

8. A program that is executed in a computer comprised in a nasal drop support device (200) that supports use of a nasal drop device (100) by a user, the program being executed to:

capture an image of the face of the user who puts the nasal drop device (100) in the nose by using a camera (210);
calculate, based on the image captured by the camera (210), a current relative angle between a reference line indicating the facial posture of the user and a reference line indicating the posture of the nasal drop device (100);
generate suitability information indicating whether or not the deviation degree between the relative angle and a target angle is within an allowable range;
notify the user of the content of the suitability information;
transmit the suitability information to the nasal drop device (100);
detect a tilt angle of the camera (210) with respect to a vertical direction as first angle information;

receive, from the nasal drop device (100), second angle information indicating a tilt angle of the nasal drop device (100) with respect to the vertical direction; the program being **characterized by** further being executed to detect, as third angle information, a tilt angle between the reference line indicating the facial posture of the user and a reference line indicating the posture of the nasal drop support device (200), by analyzing the image captured by the camera (210) and the first angle information, and calculate the relative angle based on the second angle information and the third angle information.

**Patentansprüche**

1. Nasentropfen-Unterstützungsvorrichtung (200), die so konfiguriert ist, dass sie die Verwendung einer Nasentropfenvorrichtung (100) durch einen Benutzer unterstützt, umfassend:

   eine Kamera (210), die so konfiguriert ist, dass sie ein Bild des Gesichts des Benutzers aufnimmt, der die Nasentropfenvorrichtung (100) in die Nase einführt;
   einen Prozessor (206), der so konfiguriert ist, dass er auf der Grundlage des von der Kamera (210) aufgenommenen Bildes einen aktuellen relativen Winkel zwischen einer Referenzlinie, die die Gesichtshaltung des Benutzers anzeigt, und einer Referenzlinie, die die Haltung der Nasentropfenvorrichtung (100) anzeigt, berechnet und Eignungsinformationen erzeugt, die anzeigen, ob der Abweichungsgrad zwischen dem relativen Winkel und einem Zielwinkel innerhalb eines zulässigen Bereichs liegt oder nicht;
   einen Benachrichtigungsabschnitt, der so konfiguriert ist, dass er den Benutzer über den Inhalt der Eignungsinformationen benachrichtigt;
   einen Kommunikationsabschnitt, der so konfiguriert ist, dass er die Eignungsinformationen an die Nasentropfenvorrichtung (100) überträgt; und
   einen Beschleunigungssensor (207), der so konfiguriert ist, dass er einen Neigungswinkel der Kamera (210) in Bezug auf eine vertikale Richtung als erste Winkelinformation erfasst, wobei
   der Kommunikationsabschnitt konfiguriert ist, um von der Nasentropfenvorrichtung (100) zweite Winkelinformationen zu empfangen, die einen Neigungswinkel der Nasentropfenvorrichtung (100) in Bezug auf die vertikale Richtung anzeigen; wobei die Nasentropfen-Unterstützungsvorrichtung (200) ist **dadurch gekennzeichnet, dass**
   der Prozessor (206) so konfiguriert ist, dass er als dritte Winkelinformation einen Neigungswinkel zwischen der Referenzlinie, die die Gesichtshaltung des Benutzers anzeigt, und einer Referenzlinie, die die Haltung der Nasentropfen-Unterstützungsvorrichtung (200) anzeigt, erfasst, indem er das von der Kamera (210) aufgenommene Bild und die erste Winkelinformation analysiert und den relativen Winkel auf der Grundlage der zweiten Winkelinformation und der dritten Winkelinformation berechnet.

2. Nasentropfen-Unterstützungsvorrichtung (200) gemäß Anspruch 1, ferner umfassend

   einen Speicher (202), der so konfiguriert ist, dass er im Voraus individuelle Daten, die als ein Ziel einen relativen Winkel zwischen einer Referenzlinie, die die Gesichtshaltung jedes Benutzers anzeigt, und der Referenzlinie der Nasentropfenvorrichtung (100) anzeigen, und Allzweckdaten speichert, die als ein durchschnittliches Ziel einen relativen Winkel zwischen Referenzlinien, die die Gesichtshaltungen einer Vielzahl von Benutzern anzeigen, und der Referenzlinie, die die Haltung der Nasentropfenvorrichtung (100) anzeigt, anzeigen, wobei
   der Prozessor (206) so konfiguriert ist, dass er eines von den individuellen Daten und den Allzweckdaten auswählt und den Zielwinkel aus dem Auswahlergebnis bestimmt.

3. Nasentropfen-Unterstützungsvorrichtung (200) gemäß irgendeinem der Ansprüche 1 bis 2, wobei die Nasentropfen-Unterstützungsvorrichtung (200) eine mobile Vorrichtung ist.

4. Nasentropfen-Unterstützungsvorrichtung (200) gemäß irgendeinem der Ansprüche 1 bis 3, wobei der Benachrichtigungsabschnitt so konfiguriert ist, dass er den Benutzer über den Inhalt der Eignungsinformationen unter Verwendung von Licht, Ton und/oder Vibration benachrichtigt.

5. Nasentropfensystem (1), umfassend:

   eine Nasentropfenvorrichtung (100), die so konfiguriert ist, dass sie Tröpfchen in die Nasenhöhle ausstößt; und
   die Nasentropfen-Unterstützungsvorrichtung (200) gemäß Anspruch 1, wobei
   entweder die Nasentropfen-Unterstützungsvorrichtung (200) oder die Nasentropfenvorrichtung (100) einen Erfassungsabschnitt aufweist, der konfiguriert ist, um ein Bild zu erfassen, das durch Aufnehmen eines Bildes des Gesichts des Benutzers, der die Nasentropfenvorrichtung (100) in die Nase einführt, erhalten wird.

6. Nasentropfensystem (1) gemäß Anspruch 5, wobei

   die Nasentropfen-Unterstützungsvorrichtung (200) den Erfassungsabschnitt, den Prozessor (206) und die Kamera (210) umfasst, und
   der Erfassungsabschnitt so konfiguriert ist, dass er das Bild von der Kamera (210) erfasst.

7. Unterstützungsverfahren für eine Nasentropfen-Unterstützungsvorrichtung (200), die die Verwendung einer Nasentropfenvorrichtung (100) durch einen Benutzer unterstützt, umfassend:

   Aufnehmen eines Bildes des Gesichts des Benutzers, der die Nasentropfenvorrichtung (100) in die Nase einführt, durch eine Kamera (210); und
   Berechnen, auf der Grundlage des von der Kamera (210) aufgenommenen Bildes, eines aktuellen relativen Winkels zwischen einer Referenzlinie, die die Gesichtshaltung des Benutzers anzeigt, und einer Referenzlinie, die die Haltung der Nasentropfenvorrichtung (100) anzeigt und Erzeugen von Eignungsinformationen, die anzeigen, ob der Abweichungsgrad zwischen dem relativen Winkel und einem Zielwinkel innerhalb eines zulässigen Bereichs liegt oder nicht,
   Benachrichtigung des Benutzers über den Inhalt der Eignungsinformation;
   Übertragen der Eignungsinformationen an die Nasentropfenvorrichtung (100);
   Erfassen eines Neigungswinkels der Kamera (210) in Bezug auf eine vertikale Richtung als erste Winkelinformationen;
   Empfangen, von der Nasentropfenvorrichtung (100), von zweiten Winkelinformationen, die einen Neigungswinkel der Nasentropfenvorrichtung (100) in Bezug auf die vertikale Richtung anzeigen; und wobei das Unterstützungsverfahren **dadurch gekennzeichnet ist, dass** es ferner umfasst:
   Erfassen, als dritte Winkelinformationen, eines Neigungswinkels zwischen der Referenzlinie, die die Gesichtshaltung des Benutzers anzeigt, und einer Referenzlinie, die die Haltung der Nasentropfen-Unterstützungsvorrichtung (200) anzeigt, durch Analysieren des von der Kamera (210) aufgenommenen Bildes und den ersten Winkelinformationen und Berechnen des relativen Winkels auf der Grundlage der zweiten Winkelinformation und der dritten Winkelinformation.

8. Programm, das in einem Computer ausgeführt wird, der in einer Nasentropfen-Unterstützungsvorrichtung (200) enthalten ist, die die Verwendung einer Nasentropfenvorrichtung (100) durch einen Benutzer unterstützt, wobei das Programm ausgeführt wird zum:

   Aufnehmen eines Bildes des Gesichts des Benutzers, der die Nasentropfenvorrichtung (100) in die Nase einführt, durch eine Kamera (210); und
   Berechnen, auf der Grundlage des von der Kamera (210) aufgenommenen Bildes, eines aktuellen relativen Winkels zwischen einer Referenzlinie, die die Gesichtshaltung des Benutzers anzeigt, und einer Referenzlinie, die die Haltung der Nasentropfenvorrichtung (100) anzeigt;
   Erzeugen von Eignungsinformationen, die anzeigen, ob der Abweichungsgrad zwischen dem relativen Winkel und einem Zielwinkel innerhalb eines zulässigen Bereichs liegt oder nicht,
   Benachrichtigung des Benutzers über den Inhalt der Eignungsinformation;
   Übertragen der Eignungsinformationen an die Nasentropfenvorrichtung (100);
   Erfassen eines Neigungswinkels der Kamera (210) in Bezug auf eine vertikale Richtung als erste Winkelinformationen;
   Empfangen, von der Nasentropfenvorrichtung (100), von zweiten Winkelinformationen, die einen Neigungswinkel der Nasentropfenvorrichtung (100) in Bezug auf die vertikale Richtung anzeigen; wobei das Programm **dadurch gekennzeichnet ist, dass** es ferner ausgeführt wird zum:
   Erfassen, als dritte Winkelinformationen, eines Neigungswinkels zwischen der Referenzlinie, die die Gesichtshaltung des Benutzers anzeigt, und einer Referenzlinie, die die Haltung der Nasentropfen-Unterstützungsvorrichtung (200) anzeigt, durch Analysieren des von der Kamera (210) aufgenommenen Bildes und den ersten Winkelinformationen und Berechnen des relativen Winkels auf der Grundlage der zweiten Winkelinformation und der dritten Winkelinformation.

**Revendications**

1. Un dispositif (200) d'assistance pour des gouttes nasales, configuré pour fournir une assistance à l'utilisation d'un dispositif (100) de gouttes nasales par un utilisateur, comprenant :

   un appareil (210) de prise de vues configuré pour capturer une image du visage de l'utilisateur qui met le dispositif (100) de gouttes nasales dans le nez ;
   un processeur (206) configuré pour calculer, sur la base de l'image capturée par l'appareil (210) de prise de vues, un angle relatif actuel entre une ligne de référence indiquant la posture faciale de l'utilisateur et une ligne de référence indiquant la posture du dispositif (100) de gouttes nasales, et pour générer des informations d'adéquation indiquant si le degré d'écart entre l'angle relatif et un angle cible se situe ou non dans une gamme admissible ;
   une partie de notification configurée pour informer l'utilisateur du contenu des informations d'adéquation ;
   une partie de communication configurée pour transmettre les informations d'adéquation au dispositif (100) de gouttes nasales ; et
   un capteur d'accélération (207) configuré pour détecter un angle d'inclinaison de l'appareil (210) de prise de vues par rapport à une direction verticale en tant qu'informations de premier angle,
   la partie de communication étant configurée pour recevoir, du dispositif (100) de gouttes nasales, des informations de deuxième angle indiquant un angle d'inclinaison du dispositif (100) de gouttes nasales par rapport à la direction verticale ; le dispositif (200) d'assistance pour des gouttes nasales étant **caractérisé en ce que** le processeur (206) est configuré pour détecter, en tant qu'informations de troisième angle, un angle d'inclinaison entre la ligne de référence indiquant la posture faciale de l'utilisateur et une ligne de référence indiquant la posture du dispositif (200) d'assistance pour des gouttes nasales, en analysant l'image capturée par l'appareil (210) de prise de vues et les informations de premier angle, et en calculant l'angle relatif sur la base des informations de deuxième angle et des informations de troisième angle.

2. Le dispositif (200) d'assistance pour des gouttes nasales selon la revendication 1, comprenant en outre

   une mémoire (202) configurée pour stocker à l'avance des données individuelles indiquant, en tant que cible, un angle relatif entre une ligne de référence indiquant la posture faciale de chaque utilisateur et la ligne de référence du dispositif (100) de gouttes nasales, et des données à usage général indiquant, en tant que cible moyenne, un angle relatif entre les lignes de référence indiquant les postures faciales d'une pluralité d'utilisateurs et la ligne de référence indiquant la posture du dispositif (100) de gouttes nasales,
   le processeur (206) étant configuré pour sélectionner l'une des données individuelles et des données à usage général, et déterminer l'angle cible à partir du résultat de la sélection.

3. Le dispositif (200) d'assistance pour des gouttes nasales selon l'une quelconque des revendications 1 à 2, dans lequel le dispositif (200) d'assistance pour des gouttes nasales est un appareil mobile.

4. Le dispositif (200) d'assistance pour des gouttes nasales selon l'une quelconque des revendications 1 à 3, dans lequel la partie de notification est configurée pour notifier à l'utilisateur le contenu des informations d'adéquation en utilisant au moins l'un parmi la lumière, le son et les vibrations.

5. Un système de gouttes nasales (1), comprenant :

   un dispositif (100) de gouttes nasales configuré pour éjecter des gouttelettes dans la cavité nasale ; et
   le dispositif (200) d'assistance pour des gouttes nasales selon la revendication 1, dans lequel
   l'un parmi le dispositif (200) d'assistance pour des gouttes nasales et le dispositif (100) de gouttes nasales a une partie d'acquisition configurée pour acquérir une image obtenue en capturant une image du visage de l'utilisateur qui met le dispositif (100) de gouttes nasales dans le nez.

6. Le système de gouttes nasales (1) selon la revendication 5, dans lequel

   le dispositif (200) d'assistance pour des gouttes nasales comprend la partie d'acquisition, le processeur (206) et l'appareil (210) de prise de vues, et
   la partie d'acquisition est configurée pour acquérir l'image provenant de l'appareil (210) de prise de vues.

7. Un procédé d'assistance d'un dispositif (200) d'assistance pour des gouttes nasales qui fournit une assistance à l'utilisation d'un dispositif (100) de gouttes nasales par un utilisateur, comprenant :

le fait de capturer une image du visage de l'utilisateur qui met le dispositif (100) de gouttes nasales dans le nez par un appareil (210) de prise de vues ; et

le fait de calculer, sur la base de l'image capturée par l'appareil (210) de prise de vues, un angle relatif actuel entre une ligne de référence indiquant la posture faciale de l'utilisateur et une ligne de référence indiquant la posture du dispositif (100) de gouttes nasales, et de générer des informations d'adéquation indiquant si le degré d'écart entre l'angle relatif et un angle cible se situe ou non dans une gamme admissible,

le fait d'informer l'utilisateur du contenu des informations d'adéquation ;

le fait de transmettre les informations d'adéquation au dispositif (100) de gouttes nasales ;

le fait de détecter un angle d'inclinaison de l'appareil (210) de prise de vues par rapport à une direction verticale en tant qu'informations de premier angle ;

le fait de recevoir, en provenance du dispositif (100) de gouttes nasales, des informations de deuxième angle indiquant un angle d'inclinaison du dispositif (100) de gouttes nasales par rapport à la direction verticale ; et

le procédé d'assistance étant **caractérisé en ce qu'**il comprend en outre

le fait de détecter, en tant qu'informations de troisième angle, un angle d'inclinaison entre la ligne de référence indiquant la posture faciale de l'utilisateur et une ligne de référence indiquant la posture du dispositif (200) d'assistance pour des gouttes nasales, par analyse de l'image capturée par l'appareil (210) de prise de vues et les informations de premier angle, et calculer l'angle relatif sur la base des informations de deuxième angle et des informations de troisième angle.

8. Un programme qui est exécuté dans un ordinateur compris dans un dispositif (200) d'assistance pour des gouttes nasales qui prend en charge l'utilisation d'un dispositif (100) de gouttes nasales par un utilisateur, le programme étant exécuté pour :

capturer une image du visage de l'utilisateur qui place le dispositif (100) de gouttes nasales dans le nez en utilisant un appareil (210) de prise de vues ;

calculer, sur la base de l'image capturée par l'appareil (210) de prise de vues, un angle relatif actuel entre une ligne de référence indiquant la posture faciale de l'utilisateur et une ligne de référence indiquant la posture du dispositif (100) de gouttes nasales ;

générer des informations d'adéquation indiquant si le degré d'écart entre l'angle relatif et un angle cible se situe ou non dans une gamme admissible ;

informer l'utilisateur du contenu des informations d'adéquation ;

transmettre les informations d'adéquation au dispositif (100) de gouttes nasales ;

détecter un angle d'inclinaison de l'appareil (210) de prise de vues par rapport à une direction verticale en tant qu'informations de premier angle ;

recevoir, du dispositif (100) de gouttes nasales, des informations de deuxième angle indiquant un angle d'inclinaison du dispositif (100) de gouttes nasales par rapport à la direction verticale ; le programme étant **caractérisé par le fait qu'**il est en outre exécuté pour

détecter, en tant qu'informations de troisième angle, un angle d'inclinaison entre la ligne de référence indiquant la posture faciale de l'utilisateur et une ligne de référence indiquant la posture du dispositif (200) d'assistance pour des gouttes nasales, en analysant l'image capturée par l'appareil (210) de prise de vues et les informations de premier angle, et en calculant l'angle relatif sur la base des informations de deuxième angle et des informations de troisième angle.

FIG. 1A

FIG. 1B

FIG. 2

FIG. 3

start
(nasal drop support
device)

S101
Is there
individual data?

No ← → Yes

S102
acquire
general-
purpose data

S103
acquire
individual
data

S104
determine target
angle

S105
transmit
measurement
request

S106
measure angle of
Sm-coordinate
system

S107
acquire face image

S108
recognize face

S109
calculate angle of
Sf-coordinate
system

S110
acquire angle of
Sd-coordinate
system

S111
calculate relative
angle

S112
Is it within
allowable range?
(transmit suitability
information)

No

S113
guidance

Yes

S114
receive operation
state

S115
notify operation
state

end
(nasal drop support
device)

wireless communication
processing

S121
receive
measurement
request

S122
transmit angle of
Sd-coordinate
system

S123
receive suitability
information

S124
transmit
operation state

start
(nasal drop device)

S131
idle state

S132
Has woken up?

S133
receive
measurement
request

No

Yes

S134
measure angle
of Sd-coordinate
system

S135
receive
suitability
information

No

Yes

S136
Is nasal drop
possible?

No

Yes

S139
notify that nasal
drop is possible

No

S137
Is in
automatic
mode?

S140
Is operation
detected?

No

Yes

Yes

S138
notify start of
nasal drop

S141
nasal drop
operation

S142
notify end of
nasal drop

end
(nasal drop device)

FIG. 4

droplets

105

ejection
portion

104

nasal drop device (drug solution dispenser)

100

101  UI portion

102  memory

103

ejection control
portion

106  processor
(control portion)

109  battery

108  wireless
portion

200

nasal drop support device (mobile device)

202  memory

208  wireless
portion

210  camera

206  processor
(control portion)

201  UI portion

209  battery

211  display
portion

212  input
portion

213  output
portion

FIG. 5

FIG. 6

FIG. 7

FIG. 8

droplets

**105**

**100**

nasal drop device (drug solution dispenser)

ejection portion

**104**

**101** UI portion

**102** memory

**103** ejection control portion

**106** processor (control portion)

**109** battery

**108** wireless portion

**200**

nasal drop support device (camera device)

**208** wireless portion

**210** camera

**206** processor (control portion)

**201** UI portion

**209** battery

**211** display portion

**212** input portion

**213** output portion

# FIG. 9

FIG. 10

droplets

105

100

nasal drop device (drug solution dispenser)

ejection
portion

104

101

UI portion

102

memory

103

106

107

ejection control
portion

processor

acceleration
sensor

109

110

battery

camera

# FIG. 11A

FIG. 11B

FIG. 12

droplets ↑

105

100

ejection
portion

nasal drop device (drug solution dispenser)

104

101

UI portion

102

memory

103

106

ejection control
portion

Processor
(control portion)

109

110

battery

camera

# FIG. 13

FIG. 14

41

400

server device

401
database

100

nasal drop device

300

network router
(gateway)

200

nasal drop support device
(mobile device/camera
device)

FIG. 15

400

server device

401

database

100

nasal drop device

301

wireless base station

200

nasal drop support device
(mobile device/camera
device)

# FIG. 16

## REFERENCES CITED IN THE DESCRIPTION

**Patent documents cited in the description**

- WO 2020501715 A
- WO 2017535396 A
- WO 3501222 A
- WO 2019155150 A1
- WO 2021014076 A1